# FASCICULE DE BREVET EUROPEEN

(11) **EP 0 451 213 B1**
(45) Date de publication et mention de la délivrance du brevet: **06.04.1994**
(21) Numéro de dépôt: 90902270.9
(22) Date de dépôt: 19.01.1990
(51) Int. Cl.: C07H 21/00, C07H 19/04, A61K 31/70, C07H 21/02, C07H 19/06, C07H 19/067, C07H 19/16, C07H 19/167, C07F 9/6558

(54) **DERIVES DE MOLECULES POLYHYDROXYLEES PERMETTANT L'INTRODUCTION D'AU MOINS UNE RAMIFICATION DANS UN OLIGONUCLEOTIDE**
DERIVATE VON POLYHYDROXYLIERTEN MOLEKÜLEN, WELCHE DIE EINFÜHRUNG VON MINDESTENS EINER VERZWEIGUNG IN EIN OLIGONUKLEOTID ERLAUBEN
DERIVATIVES OF POLYHYDROXYLATED MOLECULES FOR INTRODUCING AT LEAST ONE RAMIFICATION IN AN OLIGONUCLEOTIDE

(30) Priorité: 20.01.1989 FR 8900655
(43) Date de publication de la demande: 16.10.1991
(73) Titulaire: CIS BIO INTERNATIONAL, F-91000 Saclay (FR)
(72) Inventeur: TEOULE, Robert, F-38000 Grenoble (FR); ROGET, André, F-38600 Fontaine (FR); BAZIN, Hervé, F-38100 Grenoble (FR); SAUVAIGO, Sylvie, F-38000 Grenoble (FR)
(74) Mandataire: Des Termes, Monique
(86) Numéro de dépôt international: FR9000043
(87) Numéro de publication internationale: WO9008156

(56) Documents cités:
- EP-A- 0 183 374
- EP-A- 0 225 807
- WO-A-88/10264
- Necloesides & Nucleotides, Vol. & (1&2), 1987, Marcel Dekker, Inc., J.-P. Roduit et al.: "Synthesis of oligodeoxyribonucleotides containing an aliphatic amino liker arm at selected adenine bases and derivatization with biotin", pages 349-352, see the whole document

## Description

L'invention a pour objet des dérivés de molécules polyhydroxylées, par exemple de nucléosides ou de polyols, utilisables en synthèse oligonucléotidique pour former des oligonucléotides comportant au moins une ramification pouvant servir par exemple au marquage de l'oligonucléotide.

Les oligonucléotides marqués, par exemple les sondes d'acides nucléiques, constituent un outil de diagnostic très intéressant dans différents domaines tels que les maladies génétiques, les maladies infectieuses, le cancer et le typage cellulaire.

Dans les techniques de diagnostic, on utilise une sonde oligonucléotidique présentant une séquence complémentaire à celle de la cible, c'est-à-dire de l'échantillon biologique à déterminer. Ces techniques de diagnostic sont basées sur l'utilisation de l'hybridation, c'est-à-dire l'association par des liaisons hydrogène de deux brins d'acides nucléiques (ADN ou ARN) complémentaires dont l'un est la sonde et l'autre la cible. Cette réaction peut ainsi mettre en jeu deux chaînes d'ADN, une chaîne d'ADN et une chaîne d'ARN, ou bien encore deux chaînes d'ARN. Pour permettre la détection de la sonde, celle-ci comporte un élément marqueur approprié susceptible d'être détecté par des méthodes telles que les méthodes radioactives, immunoenzymatiques, colorimétriques, fluorimétriques ou luminescentes.

Les sondes oligonucléotidiques synthétiques sont préparées en liant entre eux, par des méthodes chimiques, des nucléosides par l'intermédiaire d'un groupement phosphate pour former une chaîne d'ADN (ou d'ARN). Ceci aboutit à la formation d'un polymère synthétique dans lequel les phosphates internucléotidiques relient toujours la fonction alcool secondaire en position 3' d'un dérivé de nucléoside à la fonction alcool primaire en position 5' d'un autre dérivé de nucléoside. Ainsi, lors de la synthèse oligonucléotidique, seules ces deux fonctions seront sollicitées, les autres fonctions étant protégées. Ceci va donc aboutir à la formation d'un oligonucléotide.

Les oligonucléotides marqués sont préparés suivant le même principe, le marqueur étant généralement fixé sur la base hétérocyclique d'un dérivé de nucléoside.

L'introduction dans la chaîne oligonucléotidique de ce dérivé de nucléoside se fait toujours en créant une liaison internucléotidique 3'-5'.

Les marqueurs peuvent être introduits de façon différente soit à l'extrémité 5', soit à l'extrémité 3', soit à l'intérieur d'un oligonucléotide. Mais dans tous les cas la chaîne obtenue sera linéaire.

Lorsqu'on utilise des éléments marqueurs non radioactifs, on peut préparer directement les sondes nucléotidiques marquées, bien avant leur utilisation, par des procédés biochimiques ou chimiques.

En revanche, lorsque l'élément marqueur est radioactif, l'introduction de cet élément dans la sonde doit être effectuée extemporanément.

Parmi les procédés biochimiques de production d'oligonucléotides marqués, on connaît un procédé d'incorporation de nucléotides biotinylés utilisant la terminale désoxynucléotidyl transférase comme il est décrit par WU dans Methods in Enzymology, vol. 65, 1980, p. 43-62. Cette méthode est intéressante car elle permet d'incorporer statistiquement plusieurs biotines (3 à 5 généralement) sur une sonde oligonucléotidique courte. Cependant, le marquage nécessite une étape supplémentaire après la synthèse de l'oligonucléotide. Par ailleurs, il est difficile de préparer une sonde comportant un nombre défini de marqueurs parce qu'on obtient un mélange complexe de produits de différentes tailles qu'il faudrait séparer pour avoir une sonde de point de fusion bien défini.

Parmi les méthodes chimiques, on connaît une méthode selon laquelle on fixe l'élément marqueur sur les bases de l'oligonucléotide, après synthèse et déprotection de celui-ci, comme il est décrit par Roduit et al, dans Nucleosides & Nucleotides, 6 (1 & 2), 349-352 (1987). Dans ce cas, on peut fixer plusieurs éléments marqueurs mais la sensibilité obtenue avec ces oligonucléotides marqués est encore insuffisante car on ne peut introduire qu'un nombre limité de groupements marqueurs. Chaque marqueur est introduit de façon non quantitative, ce qui implique l'obtention d'un mélange de produits dans lequel la sonde totalement marquée est minoritaire..

La présente invention a précisément pour objet de nouveaux dérivés de molécules polyhydroxylées, utilisables en synthèse oligonucléotidique, qui permettent d'introduire à l'extrémité de l'oligonucléotide un nombre important de molécules, par exemple de molécules de marquage pour accroître ainsi la sensibilité.

Selon l'invention, ce nouveau dérivé de molécule polyhydroxylée est un dérivé de nucléoside répondant à la formule : 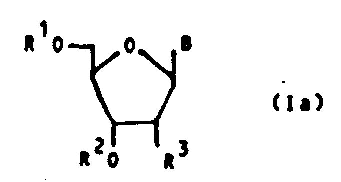
dans laquelle
- R¹ est un radical protecteur d'un groupe OH convenant à la synthèse oligonucléotidique,
- R² est un radical phosphoré adapté à la synthèse oligonucléotidique, non substitué ou substitué par OR¹,
- R³ représente un atome d'hydrogêne, le radical hydroxyle, un radical hydroxyle protégé ou le radical R⁴OR¹ avec R¹ ayant la signification donnée ci-dessus et R⁴ représentant un radical hydrocarboné bivalent, linéaire ou ramifié, substitué ou non substitué, comportant éventuellement dans sa chaîne un ou plusieurs groupements choisis parmi O, S, N H,NHCO, NHSO₂, SO₂, CO, -C≡C-, CH=CH, 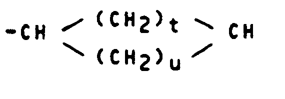 avec t et u étant des nombres entiers de 1 à 2, et 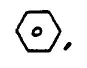 et
- B représente un radical dérivé d'une base purique ou pyrimidique répondant aux formules suivantes : 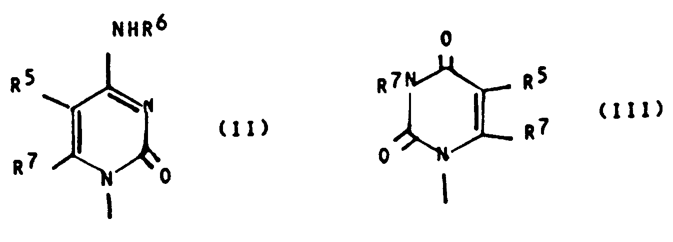 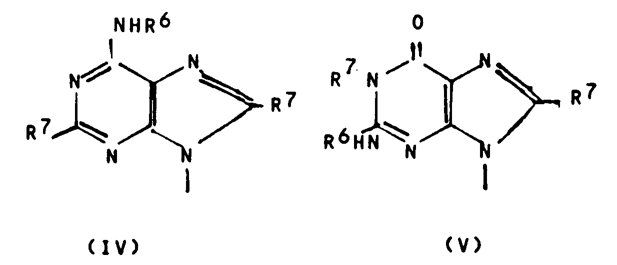 dans lesquelles R⁵ représente H, CH₃, R⁴OR¹ ou R⁸Z, R⁶ représente un groupement protecteur d'un groupe amine utilisable en synthèse nucléotidique, le radical R⁴OR¹ ou R⁸Z, R⁷ représente un atome d'hydrogène, le radical R⁴OR¹ ou R⁸Z, à condition que l'un au moins des R⁵, R⁶ et R⁷ représente R⁴OR¹ lorsque R³ ne représente pas R⁴OR¹ et que R² ne comporte pas de substituant R⁴OR¹, les R¹ et R⁴ pouvant être identiques ou différents,
- R⁴ représente un radical hydrocarboné bivalent, linéaire ou ramifié, substitué ou non substitué, comportant éventuellement dans sa chaîne un ou plusieurs groupements choisis parmi 0, S, NH, NHCO, NHSO₂, SO₂, CO, C≡C, CH=CH, 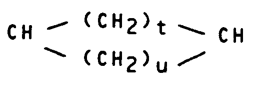 avec t et u étant des nombres entiers de 1 à 2, et 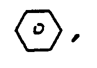
- R⁸ est une simple liaison ou un radical hydrocarboné bivalent linéaire ou ramifié, substitué ou non substitué comportant éventuellement dans sa chaîne et/ou dans ses ramifications, un ou plusieurs groupements choisis parmi O, S, NH, NHCO, NHSO₂, SO₂, CO, C≡C, CH=CH, 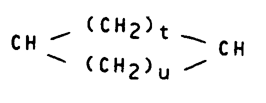 avec t et u étant des nombres entiers de 1 à 2, et 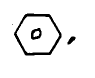 et
- Z est un radical dérivé d'une molécule de marquage d'une molécule d'un précurseur de marquage ou d'une molécule capable de s'associer à d'autres molécules, à condition que lorsque B représente le radical de formule II avec R⁶ représentant R⁴OR¹, R⁴ ne représente pas (CH₂)ₙ avec n étant un nombre entier de 1 à 6.

On précise que dans la formule donnée ci-dessus, lorsque le dérivé de nucléoside de l'invention comprend plusieurs R⁴, ceux-ci peuvent être identiques ou différents.

Comme on l'a vu précédemment, les radicaux R⁴ sont des radicaux hydrocarbonés substitués ou non substitués qui peuvent comporter de plus divers groupements.

Les substituants utilisés peuvent être en particulier des radicaux aryle, cycloalkyle, cyano, halo, nitro et acyle.

Généralement, R⁴ comprend de 1 à 20 atomes de carbone, de préférence de 3 à 10 atomes de carbone. A titre d'exemples de tels radicaux, on peut citer les radicaux de formule :
- (CH₂)ₙ-
- (CH₂)ₙ-X-(CH₂)ₚ-
- NH(CH₂)ₙ-X-(CH₂)ₚ-
- O(CH₂)ₙ-X-(CH₂)ₚ-
- S(CH₂)ₙ-X-(CH₂)ₚ-
- NHCO(CH₂)ₙ-X-(CH₂)ₚ-
- NHSO₂(CH₂)ₙ-X-(CH₂)ₚ-
- CO(CH₂)ₙ-X-(CH₂)ₚ-
- SO₂(CH₂)ₙ-X-(CH₂)ₚ-
dans lesquelles n est un nombre entier de 1 à 6, p est un nombre entier de 1 à 6, et X est une simple liaison ou représente CH₂, NHCO, O, S, NH, N(CH₂)ᵣ CH₃, C≡C, -CO-, -HC=CH-, 〉CH(CH₂)ᵣOR¹, 〉C=CH(CH₂)OR₁, 〉N-(CH₂)ᵣOR¹, 〉CH-O(CH₂)ᵣOR¹, ou 〉CH-S-(CH₂)ᵣOR¹ avec r étant égal à 0 ou étant un nombre entier de 1 à 6 et R¹ ayant la signification donnée ci-dessus.

Selon l'invention, R⁸ peut aussi représenter un radical hydrocarboné comportant divers substituants et divers groupements. Les substituants utilisés peuvent être du même type que ceux décrits ci-dessus.

De même, R⁸ comprend généralement de 1 à 20, de préférence 3 à 10 atomes de carbone.

A titre d'exemples de tels radicaux, on peut citer les radicaux de formule :
-(CH₂)ₙ-
-NH(CH₂)ₙ-Y-(CH₂)_{q}-
-NH-(CH₂)ₙ-Y-(CH₂)_{q}-NH-
-O-(CH₂)ₙ-Y-(CH₂)_{q}-
-O-(CH₂)ₙ-Y-(CH₂)_{q}-NH-
-S-(CH₂)ₙ-Y-(CH₂)_{q}-
-S-(CH₂)ₙ-Y-(CH₂)_{q}-NH-
-NHCO-(CH₂)ₙ-Y-(CH₂)_{q}-
-NHCO-(CH₂)ₙ-Y-(CH₂)_{q}-NH-
-NHSO₂-(CH₂)ₙ-Y-(CH₂)_{q}-
-NHSO₂-(CH₂)ₙ-Y-(CH₂)_{q}-NH-
-(CH₂)ₙ-Y-(CH₂)_{q}-
-(CH₂)ₙ-Y-(CH₂)_{q}-NH-
-CO-(CH₂)ₙ-Y-(CH₂)_{q}-
-CO-(CH₂)ₙ-Y-(CH₂)_{q}-NH-
dans lesquelles n est un nombre entier allant de 1 à 6, q est égal à 0 ou est un nombre entier de 1 à 6, Y représente CH₂, O, S, NH, CO, NHCO, -HC=CH-, 〉C=CH(CH₂)ᵣZ, 〉C=CH(CH₂)ᵣNHZ, 〉N(CH₂)ᵣZ, 〉N(CH₂)ᵣNHZ, 〉CH-O(CH₂)ᵣZ, 〉CH-O-(CH₂)ᵣNHZ , 〉HCS(CH₂)ᵣZ, 〉HCS(CH₂)ᵣNHZ avec r=1 à 6, et Z étant un radical dérivé d'une molécule de marquage ou d'une molécule d'un précurseur de marqueur.

Lorsque, dans les dérivés de nucléoside de formule (Ia), R³ représente un radical hydroxyle protégé, on utilise comme groupe protecteur de cet hydroxyle les groupements habituellement utilisés dans la synthèse des ribonucléotides.

Généralement, R³ représente un atome d'hydrogène.

De préférence, dans les dérivés de nucléosides de formule Ia de l'invention, la fonction R⁴OR¹ est disposée sur la partie B du dérivé de nucléoside, c'est-à-dire sur le radical dérivé de la base purique ou pyrimidique. Lorsque la base comporte un groupement NH₂ exocyclique, on peut en particulier substituer ce groupement NH₂ exocyclique par R⁴OR¹.

A titre d'exemple, B peut représenter : 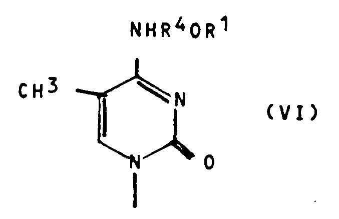
Dans ce cas, le radical R⁴ peut être choisi parmi les radicaux de formules :
- (CH₂)ₙ-X(CH₂)ₚ-
- O(CH₂)ₙ-X-(CH₂)ₚ
- S(CH₂)ₙ-X-(CH₂)ₚ-
- CO(CH₂)ₙ-X-(CH₂)ₚ-
- SO₂(CH₂)ₙ-X-(CH₂)ₚ-
dans lesquelles n, p et X ont la signification donnée ci-dessus.

A titre d'exemple, R⁴ peut représenter -(CH₂)ₙ-NH-(CH₂)ₚ avec n et p étant par exemple égaux à 2.

Dans le dérivé de nucléoside de formule (Ia) de l'invention, B est un radical dérivé d'une base purique ou pyrimidique telle que l'uracile, la thymine, la cytosine, l'adénine et la guanine. Ce radical B peut comporter différents substituants qui sont soit le radical R⁴OR¹ servant à effectuer une ramification, soit un radical protecteur des groupements NH₂ exocycliques de ces bases, soit un radical R⁸Z comportant une molécule de marquage ou une molécule d'un précurseur de marqueur.

Pour la synthèse oligonucléotidique, les groupements protecteurs des groupements NH₂ exocycliques peuvent être de différents types et sont bien connus dans la technique. Par exemple, on peut utiliser des groupes benzoyle ou anisoyle dans le cas où B est dérivé de l'adénine et de la cytosine, le groupe isobutyryle dans le cas où B est dérivé de la guanine ainsi que les groupements protecteurs décrits dans le brevet européen EP-A- 0 241 363.

De préférence, on utilise un groupement phénylpropionyle, isobutyryle ou benzoyle pour le radical de formule (II), un groupement phénoxyacétyle ou benzoyle pour le radical de formule (IV) et un groupement phénoxy acétyle ou isobutyryle pour le radical de formule (V).

Dans le cas où B comporte un groupement R⁸Z avec Z représentant une molécule de marquage ou une molécule d'un précurseur de marqueur, on peut utiliser une molécule de marquage détectable directement, par exemple des molécules capables de s'associer à l'avidine ou la streptavidine, des molécules pouvant donner lieu à une réaction du type antigène-anticorps ou des molécules ayant des propriétés fluorescentes ou luminescentes.

On peut aussi utiliser des molécules de marquage capables de recevoir un élément radioactif juste au moment de leur utilisation. En effet, lorsqu'on utilise des atomes radioactifs, il est difficile d'introduire ces éléments bien avant l'utilisation en raison de leur période et des réactions de radiolyse qu'ils induisent.

Aussi, selon l'invention, on utilise dans ce cas une molécule de marquage susceptible d'être marquée ensuite par un élément radioactif, par exemple par de l'iode radioactif.

On peut bien entendu utiliser dans le dérivé de nucléoside de l'invention d'autres molécules de marquage, en particulier celles utilisées habituellement pour la détection des antigènes, des anticorps ou des haptènes en immunologie.

Dans le cas où Z représente une molécule capable de s'associer à d'autres molécules, il peut s'agir d'une molécule capable de s'associer à une autre molécule fixée par exemple sur un support solide pour permettre l'accrochage de l'oligonucléotide sur ce support. A titre d'exemple de systèmes de ce type, on peut citer les systèmes biotine-avidine, dinitrophényl-anticorps.

Il peut s'agir également d'une molécule capable de s'associer à une autre molécule ayant une activité thérapeutique pour conférer à l'oligonucléotide une activité thérapeutique.

Les dérivés de nucléosides de l'invention peuvent être préparés par des procédés classiques tels que ceux décrits dans Organic Chemistry of Nucleic Acids. Part B.N.K. Kochetkov and E.I. Budovski, 1972 (Plenum New York). Généralement, on part des dérivés de nucléosides répondant à la formule : 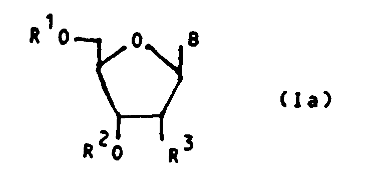
dans laquelle R¹ et R² représentent l'hydrogène, R³ représente H ou OH et B est un radical répondant aux formules II, III, IV ou V données ci-dessus avec R⁶, et R⁷ représentant un atome d'hydrogène, et R⁵ représentant un atome d'hydrogène ou un radical méthyle. On fixe ensuite sur ces dérivés les radicaux voulus par des méthodes classiques.

Lorsque le radical B du dérivé de nucléoside à préparer est un radical de formule (II), on peut aussi préparer ce dérivé en partant de la 4-thiothymidine ou de la 4-thiodésoxyuridine protégée ou non, par réaction avec une diamine, un aminoalcool, un dérivé monosubstitué de diamine ou d'aminoalcool tels que H₂N-R⁸Z ou H₂NR⁴OR', Z pouvant être une molécule de marquage dans le cas où celle-ci peut être couplée à une fonction ami ne primaire. Ceci correspond au schéma réactionnel suivant : 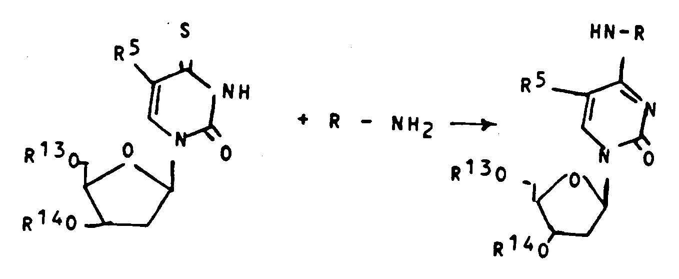
dans lequel R⁵ représente généralement H ou CH₃ et R¹³ et/ou R¹⁴, qui peuvent être identiques ou différents, représentent H ou un radical protecteur.

Après cette réaction, on peut traiter le dérivé de nucléoside pour le rendre apte à la synthèse nucléotidique et lui ajouter une ou plusieurs fonctions R⁴OR¹ ainsi qu'éventuellement des molécules de marquage du type R⁸Z.

Selon une variante de l'invention, le dérivé de molécule polyhydroxylée, est un dérivé de polyol, répondant à la formule : 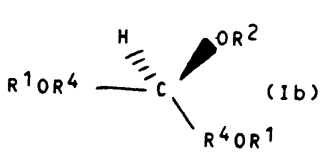
dans laquelle
- R¹ est un radical protecteur d'un groupe OH convenant à la synthèse oligonucléotidique,
- R² est un radical phosphoré adapté à la synthèse oligonucléotidique, non substitué ou substitué par OR¹,
- R⁴ représente un radical hydrocarboné bivalent, linéaire ou ramifié, substitué ou non substitué, comportant éventuellement dans sa chaîne un ou plusieurs groupements choisis parmi O, S, NH, NHCO, NHSO₂, SO₂, CO, C≡C, CH=CH, 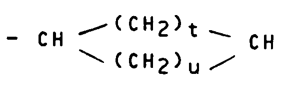 avec t et u étant des nombres entiers de 1 à 2, et

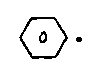

On précise également que dans la formule (Ib) donnée ci-dessus, les deux R⁴ peuvent être identiques ou différents, et peuvent représenter les mêmes radicaux que ceux décrits à propos de la formule (Ia).

A titre d'exemple, R⁴ peut représenter -(CH₂)ₙ- avec n étant un nombre entier de 1 à 6, par exemple 3.

Les dérivés de polyol de formule Ib peuvent être préparés par réduction du diester d'un acide cétodicarboxylique, de préférence symétrique. Ce critère de symétrie n'est pas absolu mais il évite la formation de diastéréoisomères et ainsi facilite la purification et l'analyse par RMN des dérivés de formule Ib fonctionnalisés en vue de leur utilisation pour la synthèse nucléotidique.

Le composé de départ est par exemple un dérivé de l'acide γ-oxopimélique.

Dans les dérivés de formules Ia et Ib de l'invention, R¹ est un radical protecteur d'un groupe OH convenant à la synthèse nucléotidique.

A titre d'exemples de radicaux R¹ susceptibles d'être utilisés, on peut citer en particulier les radicaux phényl-9-xanthényle et les radicaux trityle de formule : 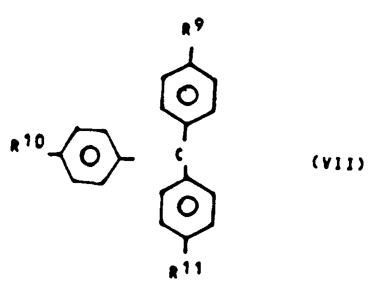
dans laquelle R⁹, R¹⁰ et R¹¹ qui peuvent être identiques ou différents, représentent un atome d'hydrogène, un radical alkyle, alcoxy ou amino. On peut aussi utiliser pour R¹ le radical t-butyl diméthyl silyl.

De préférence, R¹ est le radical méthoxy ou diméthoxy trityle, soit le radical répondant à la formule VII précitée dans laquelle R⁹ ou R⁹ et R¹⁰ représentent des groupes méthoxy, R¹¹ et éventuellement R¹⁰ représentant un atome d'hydrogène.

Selon l'invention, le radical R² est un radical fonctionnel adapté à la synthèse nucléotidique, par exemple un radical phosphoré comportant éventuellement un substituant de formule R¹⁰.

A titre d'exemples de radicaux phosphorés susceptibles d'être utilisés, on peut citer les radicaux de formules : 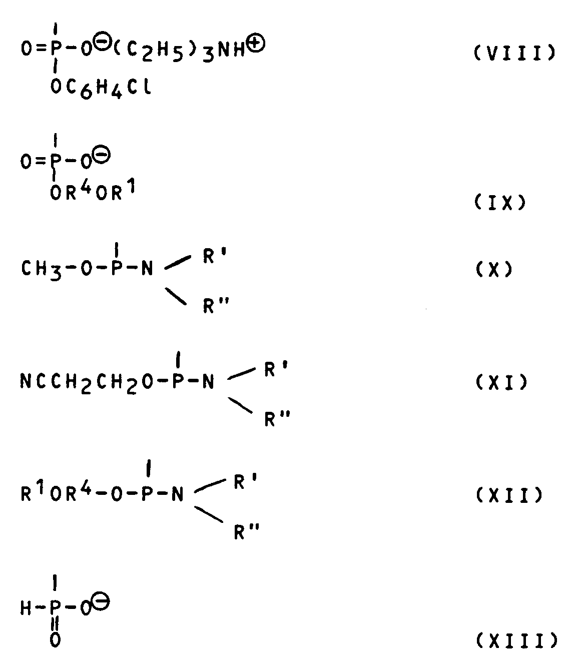
dans lesquelles R¹ a la signification donnée ci-dessus, R⁴ représente un radical hydrocarboné bivalent linéaire ou ramifié, substitué ou non, comportant éventuellement dans sa chaîne un ou plusieurs groupements O, S, NH, NHCO, NHSO₂, SO₂, CO, C≡C, CH=CH, 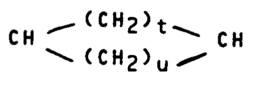
avec t et u étant des nombres entiers de 1 à 2, 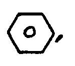
et R' et R'' pris séparément représentent chacun un radical alkyle, aralkyle ou cycloalkyle, contenant jusqu'à 10 atomes de carbone, ou R' et R'' forment ensemble une chaîne alkylène contenant jusqu'à 5 atomes de carbone dans la chaîne principale et un total allant jusqu'à 10 atomes de carbone, ou forment ensemble avec l'atome d'azote un hétérocycle saturé qui peut contenir un ou plusieurs autres hétéroatomes choisi parmi N, O et S.

De préférence, lorsque R' et R'' sont pris séparément, ils représentent des groupes alkyle inférieur comme les radicaux isopropyle, t-butyle, isobutyle, sec-butyle, néopentyle, tert-pentyle, isopentyle, sec-pentyle.

De préférence, lorsque R' et R'' forment ensemble avec l'atome d'azote un hétérocycle, cet hétérocycle est la pyrrolidine, la morpholine ou la pipéridine.

A titre d'exemple, R² représente le radical de formule : 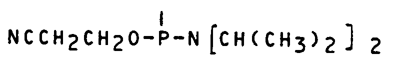
Les dérivés de molécules polyhydroxylées de formules Ia et Ib de l'invention sont très intéressants à utiliser dans une synthèse oligonucléotidique car ils permettent de créer à l'endroit voulu de l'oligonucléotide des ramifications qui peuvent être formées d'une ou plusieurs molécules de types très variés.

En effet, lorsqu'on utilise ces dérivés de molécules polyhydroxylées en synthèse oligonucléotidique, la présence de la fonction OR² permet de fixer le dérivé sur un oligonucléotide déjà formé et la présence de 2 à 4 fonctions OR¹ permet de poursuivre la synthèse en effectuant des ramifications, c'est-à-dire en fixant ensuite sur ce dérivé 2 à 4 molécules capables de réagir avec les fonctions OH obtenues par coupure des liaisons OR¹.

Les molécules fixées peuvent être de différents types.

A titre d'exemples de telles molécules, on peut citer les nucléosides, les nucléosides marqués, les molécules pouvant servir d'espaceurs, les molécules de marquage, les molécules capables d'être marquées, et les molécules capables de s'associer à d'autres molécules pour conférer à l'oligonucléotide des propriétés particuliéres, par exemple réaliser son accrochage sur un support solide ou le rendre thérapeutiquement actif. On peut aussi utiliser des dérivés de molécules polyhydroxylées conformes à l'invention pour réaliser d'autres ramifications.

En choisissant de façon appropriée les molécules utilisées, on peut aussi poursuivre la synthèse en effectuant des ramifications formées chacune d'une succession de molécules identiques ou différentes en vue de conférer à l'oligonucléotide des propriétés intéressantes, par exemple pour sa détection.

Les dérivés de molécules polyhydroxylées de l'invention présentent donc un grand intérêt en synthèse oligonucléotidique.

Aussi, l'invention a également pour objet un procédé pour former au moins une ramification sur un oligonucléotide qui comprend les étapes successives suivantes :
a) fixation sur cet oligonucléotide d'un dérivé de molécule polyhydroxylée de formule : 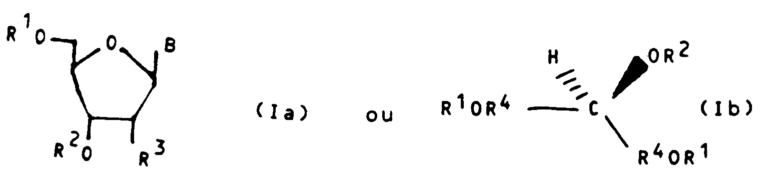 dans lesquelles R¹, R², R³, R⁴ et B ont la signification donnée ci-dessus,
b) réaction des fonctions OH du dérivé fixé avec des molécules capables de réagir avec les fonctions OH obtenues par coupure des liaisons OR¹.

Selon une variante de mise en oeuvre de ce procédé, on fixe tout d'abord sur l'oligonucléotide une molécule servant d'espaceur, puis on fixe sur cette molécule servant d'espaceur le dérivé de l'invention et on fixe ensuite sur ce dérivé des molécules capables de réagir avec les fonctions OH générées par coupure des OR¹.

Les molécules fixées sur le dérivé de l'invention peuvent être de différents types du moment qu'elles possèdent une fonction capable de réagir avec les fonctions OH obtenues par coupure des OR¹ du dérivé de l'invention.

A titre d'exemples, ces molécules peuvent être des nucléosides, des nucléosides marqués, des molécules pouvant servir d'espaceurs (molécules qui comportent non seulement une fonction capable de réagir avec la fonction OH obtenue par coupure de OR¹, mais aussi une fonction permettant la fixation d'une autre molécule, par exemple d'un nucléoside), des molécules de marquage, des molécules pouvant être marquées, des molécules capables de s'associer à d'autres molécules ou d'autres dérivés de molélcules polyhydroxylées conformes à l'invention.

L'utilisation des dérivés de l'invention en synthèse oligonucléotidique permet ainsi de réaliser des structures et des configurations très variées.

A titre d'exemples non limitatifs, on peut réaliser les structures suivantes dans lesquelles :
- N représente le dérivé de l'invention,
- N' représente une molécule servant d'espaceur,
- M représente une molécule marquée ou susceptible d'être marquée, ou encore une molécule capable de s'associer à d'autres molécules.

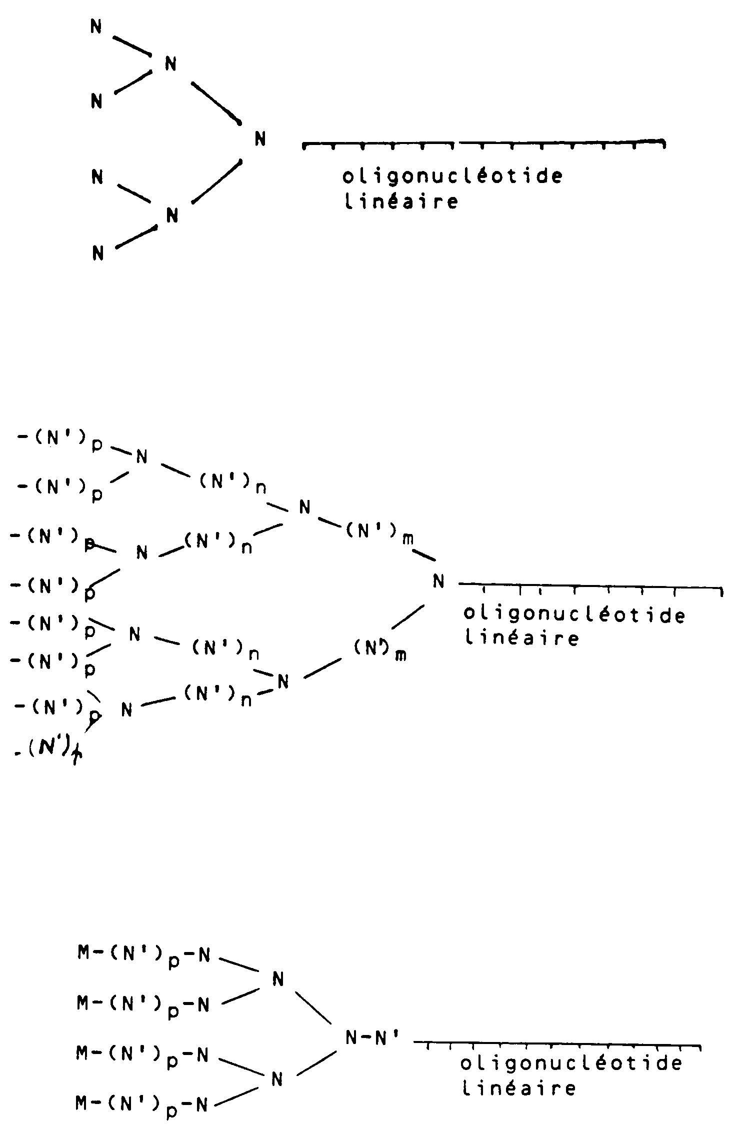

Dans ces structures, les molécules N' servant d'espaceurs peuvent être des chaînes hydrocarbonées comportant à leurs extrémités des fonctions appropriées leur permettant d'être intercalées entre des molécules N, N' et/ou M.

Les molécules N' servant d'espaceurs peuvent aussi être constituées par des dérivés de nucléosides, par exemple la désoxythymidine.

L'introduction de ces dérivés de nucléosides N', qui servent uniquement d'espaceurs, permet d'améliorer la solubilité dans l'eau et le milieu biologique de l'oligonucléotide obtenu, d'écarter les marqueurs de la séquence d'hybridation (oligonucléotide linéaire), et d'assurer ainsi une meilleure accessibilité des marqueurs éventuellement fixés au bout des ramifications.

Les molécules M de marquage peuvent également être de différents types.

Ainsi, on peut utiliser une molécule de marquage détectable directement, par exemple des marqueurs fluorescents ou phosphorescents, des molécules antigéniques ou des dérivés ayant une forte affinité pour d'autres molécules.

A titre d'exemples, les molécules de marquage directement détectables peuvent être des molécules capables de s'associer à l'avidine ou la streptavidine, des molécules pouvant donner lieu à une réaction du type antigène-anticorps ou des molécules ayant des propriétés fluorescentes ou luminescentes.

On peut aussi utiliser des molécules de marquage capables de recevoir un élément radioactif juste au moment de leur utilisation. En effet, lorsqu'on utilise des atomes radioactifs, il est difficile d'introduire ces éléments bien avant l'utilisation en raison de leur période et des réactions de radiolyse qu'ils induisent. Aussi, selon l'invention, on utilise dans ce cas une molécule de marquage susceptible d'être marquée ensuite par un élément radioactif par exemple par de l'iode radioactif.

Ces molécules de marquage peuvent être introduites directement ou par l'intermédiaire d'un dérivé de nucléoside les comportant, par exemple d'un dérivé de nucléoside de formule (Ia) comportant un radical R⁸Z et une ou plusieurs fonctions OR¹.

On peut encore utiliser comme molécule M susceptible d'être marquée, un dérivé de nucléoside naturel que l'on marque ensuite par un élément radioactif tel que le ³²P, par exemple en faisant réagir la fonction hydroxyle de ce dérivé de nucléoside avec un phosphate radioactif, par exemple par action de γ ³²P ATP et de la polynucléotide kinase.

Ainsi, avec les structures d'oligonucléotides obtenues selon l'invention, il est possible d'avoir une densité importante de molécules de marqueurs à l'extrémité de l'oligonucléotide et d'obtenir un signal très sensible par marquage au phosphore 32.

Selon les molécules utilisées, on peut ainsi obtenir des oligonucléotides ayant les propriétés voulues, par exemple des oligonucléotides marqués ayant une sensibilité élevée.

La molécule M peut encore être une molécule capable de s'associer à d'autres molécules, par exemple pour réaliser l'accrochage de l'oligonucléotide sur un support solide ou le rendre thérapeutiquement actif, comme on l'a vu précédemment.

L'utilisation des dérivés de l'invention en synthèse oligonucléotidique ne pose pas de problème car ils peuvent être utilisés directement comme d'autres synthons dans les appareils automatiques de synthèse oligonucléotidique, et ils peuvent subir sans altération toutes les opérations de la synthèse oligonucléotidique. De plus, ils ont des réactivités proches de celles des synthons commerciaux utilisés actuellement.

Pour la synthèse oligonucléotidique, on peut utiliser la méthode de synthèse du phosphotriester lorsque R² est le radical de formule (VIII) ou (IX), la méthode de synthèse du phosphoramidite dans le cas où R² est le radical de formules X, XI ou XII, ou la méthode de synthèse des phosphonates dans le cas où R² est le radical de formule (XIII).

La synthèse peut être effectuée soit par des méthodes en solution, soit par des méthodes de synthèse sur support. On utilise de préférence les méthodes de synthèse sur support qui donnent actuellement les meilleurs résultats.

La présente invention a encore pour objet les oligonucléotides obtenus par le procédé décrit ci-dessus.

Ces oligonucléotides répondent à la formule :

Aₙ₁N'ₐNC

dans laquelle :
- n1 est un nombre entier supérieur à 1,
- les A sont des nucléotides identiques ou différents,
- N' est une molécule servant d'espaceur,
- a est égal à 0 ou est un nombre entier,
- N est un dérivé de molécule polyhydroxylée conforme à l'invention, et
- C représente l'extrémité de l'oligonucléotide qui comprend au moins deux ramifications identiques ou différentes, formées chacune d'une ou plusieurs molécules identiques ou différentes, choisies parmi N', N, M et A avec M représentant un nucléotide marqué, une molécule de marquage, une molécule d'un précurseur de marqueur ou une molécule capable de s'associer à d'autres molécules.

D'autres caractéristiques et avantages de l'invention apparaîtront mieux à la lecture des exemples suivants donnés bien entendu à titre illustratif et non limitatif.

### Exemple 1 : Préparation du composé n° 1 de formule :

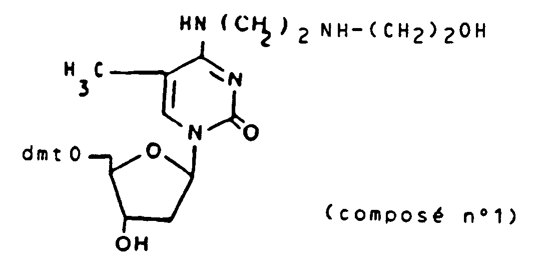

Ce composé n°1 est un dérivé de nucléoside de formule (Ia) dans laquelle R¹ représente le radical diméthoxytrityle (dmt), R² et R³ représentent un atome d'hydrogène, B représente le radical de formule (II) avec R⁵ représentant CH₃, R⁷ représentant un atome d'hydrogène et R⁶ représentant le radical R⁴OR¹ avec R⁴ représentant (CH₂)₂NH(CH₂)₂ et R¹ représentant H.

On dissout 840mg (1,5mmol) de 5'-diméthoxytrityl, 4-thiothymidine dans 10ml d'isopropanol et 610µm (4mmol) de N-(2-hydroxyéthyl)-éthylène diamine et on chauffe pendant 16h dans un flacon bouché à 60°C. Après évaporation à sec, on reprend le mélange par 25ml de chloroforme et on le lave 3 fois avec 50ml de bicarbonate de sodium à 5% et 50ml d'eau distillée. Après évaporation du solvant organique, le produit est cristallisé dans du toluène et est obtenu avec un rendement de 71%.

### Exemple 2 : Préparation du composé n° 2 de formule :

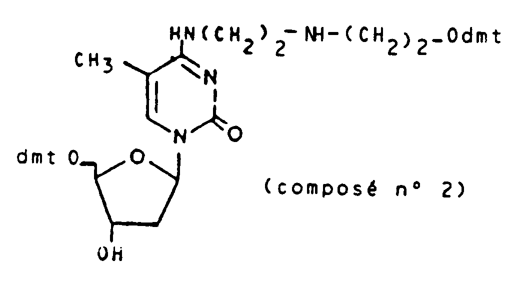

On sèche 296mg (0,5mmol) du composé n° 1 par coévaporation dans de la pyridine anhydre. On ajoute 1,2éq. de chlorure de diméthoxytrityle et on laisse le mélange réactionnel pendant 16h à 0°C. Après arrêt de la réaction par 1ml de méthanol, on ajoute 50ml d'une solution de bicarbonate de sodium à 5% et 50ml de chloroforme.

Après décantation, on lave la phase organique deux fois par 50ml de bicarbonate, puis une fois par 50ml d'eau distillée. On contre-extrait les phases aqueuses par 50ml de chloroforme. On réunit les phases organiques et on les évapore à sec. On sépare le produit sur colonne de silice en éluant par un gradient d'éthanol dans le chloroforme (0 à 20%). On obtient ainsi le composé n° 2 avec un rendement de 73%.

**Exemple 3** : Préparation du composé n° 3 de formule : 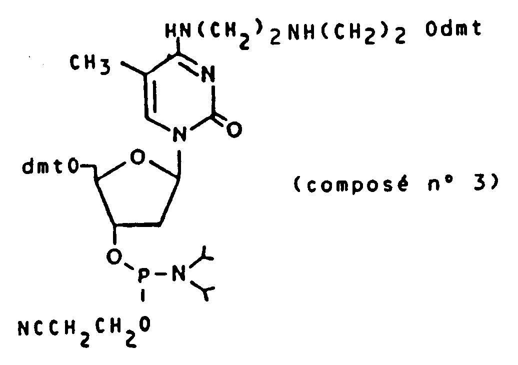
On sèche par coévaporation dans un mélange de dichlorométhane et d'acétonitrile anhydre 472mg (0,5mmol) du composé n° 2,puis on le redissout dans 5ml de dichlorométhane anhydre. On ajoute 0,25mmol de tétrazolate de diisopropylammonium et 0,6mmol de bis-diisopropylaminocyanoéthoxyphosphine. On laisse la réaction se poursuivre pendant 2h à la température ambiante. On dilue le mélange réactionnel à 25ml, puis on le lave 3 fois par 25ml de NaHCO₃ en solution aqueuse saturée. On évapore sous vide la phase organique, on reprend le résidu par 5ml de solution chloroformique et on le précipite dans 100ml d'hexane refroidi à -80°C. On obtient ainsi le composé n° 3 avec un rendement de 94%.

### Exemple 4 : Préparation du composé n° 4 de formule :

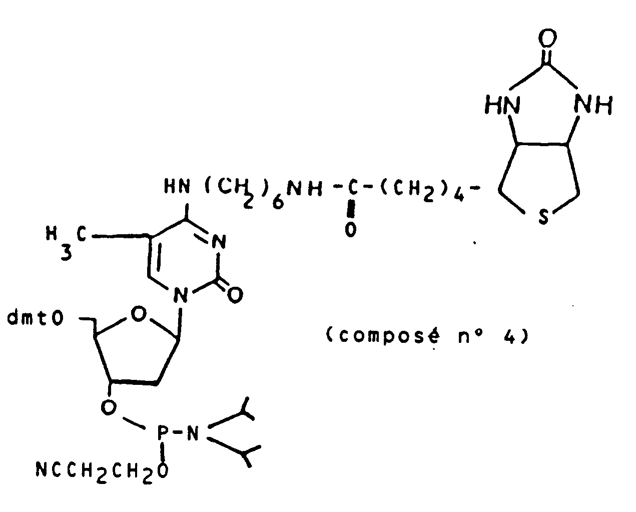

### a) Préparation du composé n° 5 de formule :

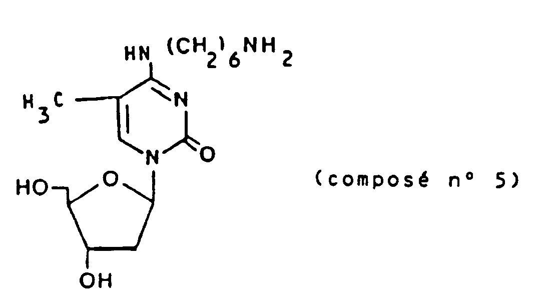

On dissout 1mmol de 4-thiothymidine dans 5ml d'éthanol absolu, et on y ajoute 5mmol de 1,6-diaminohexane, et on maintient pendant 16h à 60°C. On évapore à sec, on reprend par 10ml d'eau et 0,2ml de triéthylamine et on évapore. On triture le résidu par 4 fois 10ml d'éther éthylique. On adsorbe le résidu sur 1g de Célite 545 et on le dépose sur une colonne de Célite préparée avec la phase inférieure du mélange acétate d'éthyle-méthoxyéthanol-eau 4:1:2, on élue la colonne par la phase supérieure de ce mélange, puis on élue le produit recherché par la phase supérieure du mélange acétate d'éthyle-n-butanol-eau 1:1:1. Par évaporation des fractions appropriées et lyophilisation, on obtient ainsi le composé n° 5.

### b) Préparation du composé n° 6.

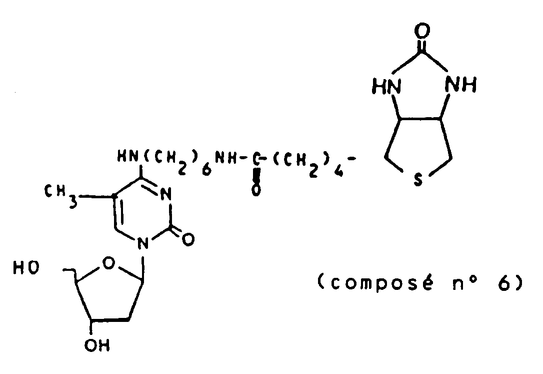

On dissout 681mg (2mmol) du composé n° 5 dans 15ml de diméthylformamide et on ajoute 0,4ml (4mmol) de triéthylamine et 700mg (2mmol) de N-hydroxysuccinimidobiotine. Après 3h sous agitation à 20°C, on évapore sous vide le mélange réactionnel, puis on le coévapore avec 2 fois 15ml d'eau distillée puis avec 2 fois 20ml d'éthanol absolu. On reprend le résidu par du méthanol, on ajoute 8g de gel de silice (0,2 à 0,5mm) et on sèche la suspension sous vide. On dépose alors sur une colonne de gel de silice (gel 60 de Merck) le mélange adsorbé préalablement sur la silice, et on élue ensuite la colonne par un gradient de méthanol dans le chloroforme (5 à 20%).

On obtient ainsi 715mg du composé n°6, ce qui correspond à un rendement de 63%.

### c) Préparation du composé n° 7 de formule :

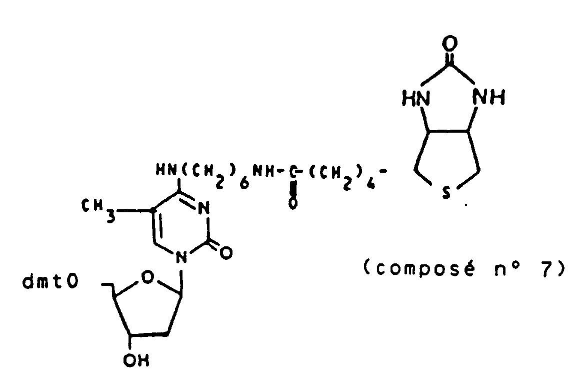

On prépare ce composé à partir du composé n° 6 de la façon suivante.

On dissout à chaud 1,14g (2mmol) du composé n° 6 dans de la pyridine anhydre, on refroidit la solution et on l'évapore sous vide. On reprend le résidu par 5ml de diméthylformamide anhydre et on dilue par 20ml de pyridine anhydre, puis on refroidit le mélange à 0°C sous agitation. On ajoute alors 744mg de chlorure de diméthoxy-4,4'-trityle. On maintient le mélange pendant 16h sous agitation. On ajoute alors 2ml de méthanol, puis après 15min on verse le mélange réactionnel sur 35ml d'une solution aqueuse saturée de bicarbonate de sodium.

On extrait le mélange par deux fois 50ml de chloroforme, puis on évapore la phase organique et on la coévapore avec deux fois 20ml de toluène.

On reprend le résidu par du dichlorométhane et on le purifie par chromatographie sur gel de silice (gel 60 de Merck) en utilisant pour l'élution un gradient de méthanol dans du dichlorométhane.

On obtient ainsi 1,44g du composé n° 7, ce qui correspond à un rendement de 83%.

### d) Préparation du composé n° 4.

On rend anhydre 870mg (1mmol) du composé n°7 par coévaporation dans la pyridine absolue, puis on redissout dans 10ml de dichlorométhane anhydre. On ajoute 85mg (0,5mmol) de tétrazolate de diisopropylammonium ainsi que 340µl de bis(diisopropylamino)cyanoéthoxyphosphine. On agite le milieu réactionnel pendant 2h à la température ambiante, puis on ajoute 50ml de dichlorométhane. On lave la solution organique par trois fois 50ml de bicarbonate de sodium en solution aqueuse saturée, puis par 50ml d'une solution saturée de chlorure de sodium.

On évapore sous vide la phase organique, on reprend le résidu par 5ml de dichlorométhane et on le précipite dans 100ml d'hexane refroidi à -78°C. On collecte le précipité et on sèche sous vide.

On obtient ainsi 930mg du composé n° 4, ce qui correspond à un rendement de 87%.

### Exemple 5 : Préparation d'un oligonucléotide ramifié.

Cet exemple illustre la préparation d'un oligonucléotide ramifié marqué à la biotine en utilisant les composés n° 3 et 4.

L'oligonucléotide a la séquence suivante : 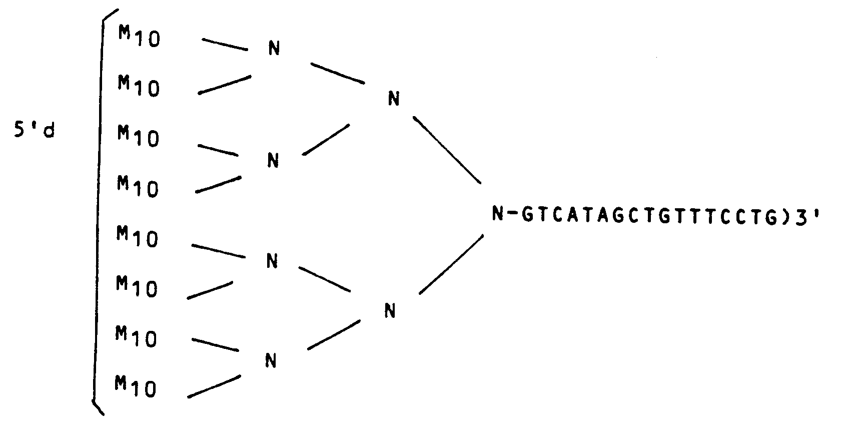
Dans cette séquence, A représente le nucléotide formé par l'adénine, C représente le nucléotide formé par la cytosine, G représente le nucléotide formé par la guanine, T représente le nucléotide formé avec la thymine, N représente le nucléotide formé à partir du composé n° 3 conforme à l'invention, et M représente le nucléotide formé à partir du composé n° 4.

Pour effectuer cette synthèse, on utilise le composé n° 3 comme synthon correspondant à N et le composé n° 4 comme synthon correspondant à M. Les autres synthons utilisés sont des synthons correspondant à l'adénine, à la cytosine, à la guanine et à la thymine qui sont obtenus à partir des nucléosides correspondants en protégeant la fonction hydroxyle en 5' par le radical dmt et en fonctionnalisant le fonction hydroxyle en 3' par le radical de formule : 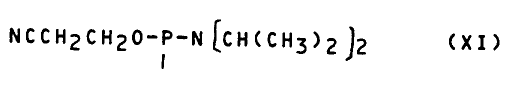
Pour les nucléotides correspondant à l'adénine et à la cytosine, la fonction aminée exocyclique est protégée par un groupement benzoyle. Pour les nucléotides correspondant à la guanine, elle est protégée par le groupement isobutyrile.

La synthèse a été effectuée au moyen d'un synthétiseur automatique d'oligonucléotides Applied Biosystems 381 A en utilisant pour la synthèse de l'oligonucléotide linéaire :
- 0,2µmoles de nucléoside greffé sur un support "Controlled Pore Glass" comportant un bras espaceur forme par une chaîne aminée, la liaison entre ce nucléoside et l'amine étant réalisée par un radical succinyle.
- 10mg (40 eq) par cycle de condensation des synthons de désoxy-2'-adénosine, de désoxy-2'-cytidine, de désoxy-2'-guanosine et de thymidine, soit environ 40 équivalents par rapport au support.
- 7mg par cycle de condensation d'un agent d'activation constitué par du tétrazole, soit environ 10 équivalents par rapport au nucléotide.

Le cycle de condensation est le cycle standard "0,2µmol cyanoéthylphosphoramidite"de l'appareil Applied Biosystems 381 A qui comprend une étape préalable de détritylation, c'est-à-dire de coupure des liaisons OR¹ pour les transformer en OH.

A la fin du cycle de condensation, on obtient l'oligonucléotide linéaire protégé lié de façon covalente au support par l'intermédiaire du bras espaceur succinylé.

On fixe ensuite sur l'oligonucléotide linéaire le nucléotide correspondant au synthon formé par le composé n° 3 en utilisant également 10mg du composé n° 3, soit 40éq., puis on procède au premier cycle de ramification en utilisant 20mg, soit 80méq. du composé n° 3, puis au deuxième cycle de ramification, en utilisant 40mg, soit 160éq. du composé n° 3.

On effectue ensuite dix cycles de condensation pour fixer au bout de chaque ramification, 10 nucléotides correspondant au composé n° 4.

Pour les cycles de condensation faisant intervenir le composé n° 3 ainsi que pour les 10 cycles de condensation faisant intervenir le composé n° 4, on utilise le cycle standard "1µmol".

L'agent de condensation utilisé est le tétrazole. Le temps de réaction est de 3min pour les couplages faisant intervenir le composé n° 3 et le composé n° 4.

En fin d'opération, l'oligonucléotide est coupé du support par traitement à l'ammoniaque à 28% pendant 2h, puis la solution ammoniacale obtenue est chauffée 5 à 16h à 60°C pour éliminer les groupements protecteurs. L'ammoniaque est évaporé, le résidu est repris par 500µl d'eau distillée et filtré sur une colonne de Sephadex G25 (diamètre 1cm, hauteur 7cm), les fractions correspondant au premier pic sont lyophilisées. Le produit est séparé par électrophorèse sur gel d'agarose.

### Exemple 6 :

On prépare de la même façon que dans l'exemple 5, un oligonucléotide dont la séquence est la suivante : 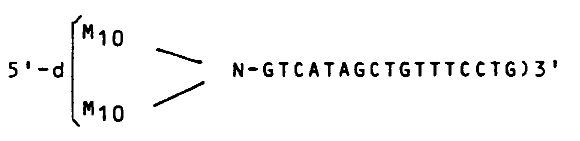
en utilisant les mêmes synthons. On obtient ainsi un oligonucléotide comportant en bout de chaîne 20 nucléotides biotynilés à son extrémité 5'.

### Exemple 7.

On prépare de la même façon que dans l'exemple 5 un oligonucléotide dont la séquence est la suivante : 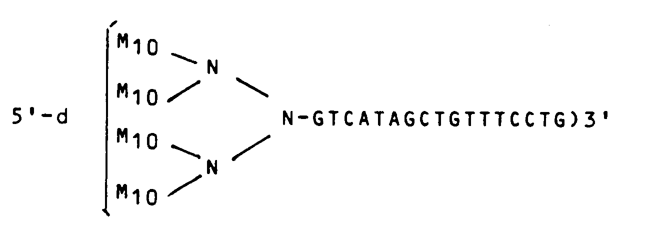
On obtient ainsi un oligonucléotide comportant 40 nucléotides marqués à l'extrémité de la séquence.

### Exemple 8.

On prépare de la même façon que dans l'exemple 5 un oligo nucléotide comportant la séquence suivante : 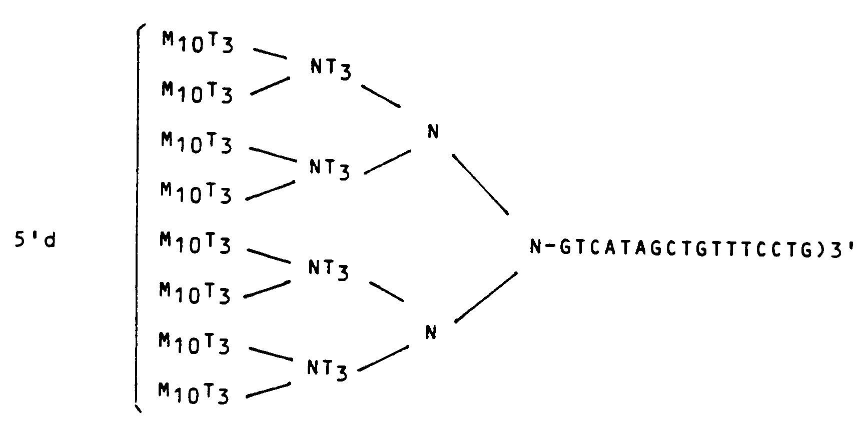
dans laquelle T représente la thymidine, trois thymidines étant intercalées dans les ramifications entre deux dérivés de nucléosides de l'invention pour servir d'espaceur.

On obtient ainsi un oligonucléotide comportant 80 dérivés de nucléosides marqués à la biotine à son extrémité 5'.

### Exemple 9 : Préparation d'un polyol répondant à la formule (Ib) avec R₄=(CH₂)ₙ avec n=3 et R₁=R₂=H.

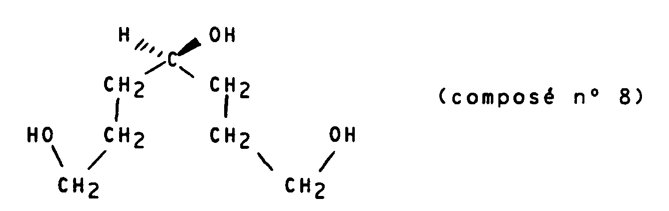

On ajoute 250mg d'hydrure double de lithium aluminium à du 3-cétopimelate d'éthyle tel que celui décrit par Rabjohn, N, Org. Synth, col. vol. 4 (1963) 302 (500mg, 2,2mmol) dans le tétrahydrofurane anhydre (40ml) refroidi à 0°C. Après 15min, on porte à reflux pendant 15 à 20min puis on laisse refroidir progressivement. On refroidit à 0°C et on ajoute environ 5ml d'une solution à 10% d'eau dans le tétrahydrofurane.

On laisse 30min sous agitation, on filtre le précipité d'aluminates et on le lave par 3x10ml de tétrahydrofurane. On évapore le filtrat et on soumet le résidu à la chromatographie sur colonne courte (silice G Merck) en utilisant un mélange chloroforme-méthanol 95:5 puis 85:15.

On obtient 300mg d'un liquide visqueux.

### Exemple 10 : Préparation d'un polyol partiellement protégé répondant à la formule (Ib) avec R₄=(CH₂)ₙ avec n=3, R₁=dmt et R₂=H.

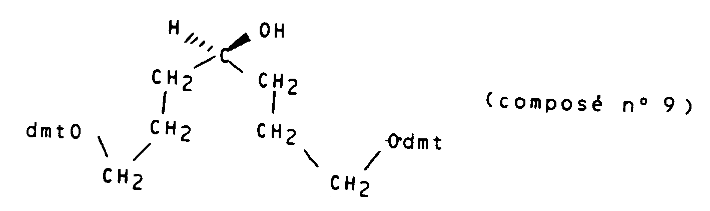

On ajoute 20ml de pyridine et 1,2 équivalent de chlorure de diméthoxy trityle à 300mg (2,2mmol) du composé n°8 co-évaporé en présence de pyridine anhydre. On Laisse le mélange sous agitation pendant 3h à 0°C. On ajoute alors 10ml d'une solution aqueuse de bicarbonate de sodium à 5% et après 30min on effectue une partition entre chloroforme et eau. La phase organique est évaporée et le résidu est chromatographié sur une colonne de silice G (Merck) en éluant par un gradient (30 à 100%) de dichlorométhane dans l'hexane puis par 2% d'acétone dans le dichlorométhane.

On obtient 1,08g (72%) du composé n° 9.

### Exemple 11 : Préparation du composé n° 10 correspondant à la formule (Ib) avec R₄=(CH₂)ₙ avec n=3, R₁=dmt et R₂=le radical de formule (XI) :

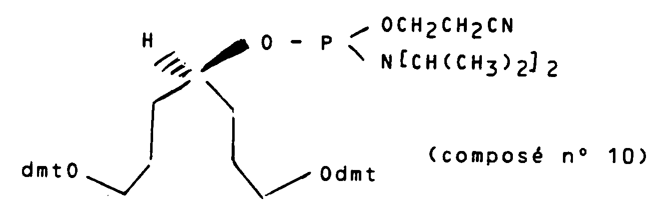

Le composé n° 9 est traité par la bis-diisopropylaminocyanoéthoxyphosphine suivant la méthode décrite dans l'exemple 3.

Après traitement, on purifie le produit par chromatographie sur colonne courte avec un gradient de dichlorométhane dans l'hexane, suivie d'une précipitation dans l'hexane à -78°C.

On obtient ainsi le composé n° 10.

### Exemple 12 : Préparation du composé n° 11 de formule :

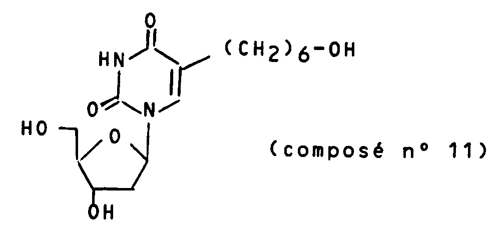

Ce composé n° 11 est un dérivé du nucléoside de formule (Ia) dans laquelle R₁, R₂ et R₃ représentent un atome d'hydrogène, B représente le radical de formule (III) avec R₅ représentant R₄OR₁ avec R₄ représentant (CH₂)₆ et R₁ représentant un atome d'hydrogène et R₇ représente un atome d'hydrogène.

Le produit de départ, la 5-chloromercuri-2'-désoxyuridine, est préparé selon Bergstrom et al. (J. Carbohydr., Nucleoside Nucleotides 1977, 4, 257-269).

Une solution (0,1M) de tétrachloropalladate de lithium (11ml) dans le méthanol est ajoutée à une suspension de 0,5g de 5-chloromercuri-2'-désoxyuridine dans du méthanol (10ml). On ajoute 0,33ml d'hexène-5-ol-1 et on porte à reflux pendant 16h. Le mélange réactionnel est refroidi puis saturé avec H₂S et filtré sur célite. Le filtrat est évaporé et adsorbé sur du silicagel (0,2-0,5mm) par coévaporation avec un mélange chloroforme-méthanol. Le silicagel est déposé sur une colonne courte de gel de silice G (Merk). Le produit est élué avec un gradient de méthanol dans le chloroforme (0 à 20%).

Par évaporation on obtient 0,3g de composé n° 12 qui est soumis à une hydrogénation catalytique. 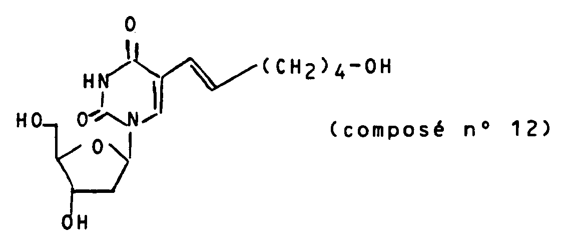
Le composé ci-dessus est dissous dans 20ml de méthanol et hydrogéné en présence de 30mg de palladium sur carbone à 10%. Après 6h d'hydrogénation (30 PSI soit 2 atm.), le catalyseur est filtré et le solvant est évaporé. On obtient 0,25g (80%) du composé n° 11.

### Exemple 13 : Préparation du composé n° 13 de formule :

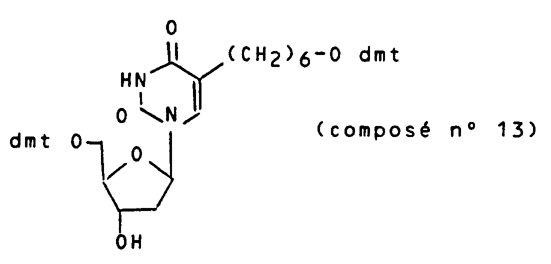

Le composé n° 11 (0,25g, 0,8mmol) est traité par 0,6g de chlorure de diméthoxytrityle dans 15ml de pyridine en suivant le protocole décrit pour le composé n° 2 dans l'exemple 2. On obtient 0,31g de composé n° 13.

### Exemple 14 : Préparation du composé n° 14 de formule :

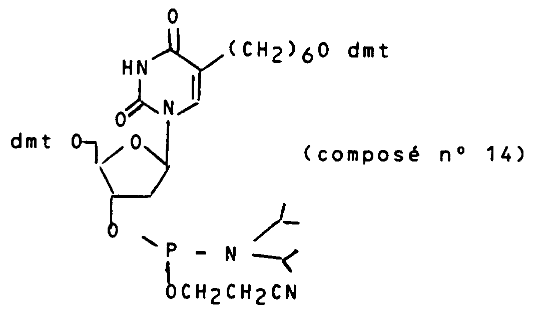

Le composé n° 13 est phosphitylé en utilisant le procédé décrit dans l'exemple 3 pour la préparation du composé n° 3.

Après précipitation dans l'hexane, on obtient le composé n° 14.

### Exemple 15 : Préparation du composé n° 15 de formule :

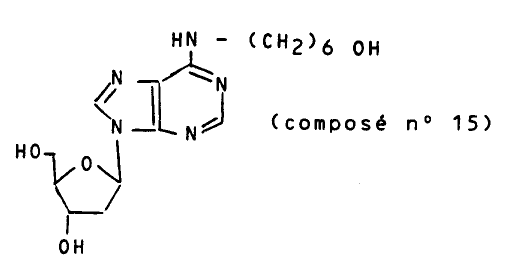

Ce composé n° 15 est un dérivé du nucléoside de formule (Ia) dans laquelle R₁, R₂ et R₃ représentent des atomes d'hydrogène, B représente le radical de formule (IV) avec R₇ représentant un atome d'hydrogène, R₆ représente le radical R₄OR₁ avec R₄ représentant (CH₂)₆ et R₁ représentant un atome d'hydrogène.

Le produit de départ la 6-chloro-9-(2'-désoxyribofuranosyl)-purine est préparé selon Z. Kazimierczuk et al. J. Am. Chem. Soc., 106, 6379 (1984).

Le 6-chloronucleoside (500mg) est traité par de l'amino-6-hexanol-1 (300mg, 2,5eq). On porte 3h à reflux puis on évapore à sec et on reprend par 15ml d'eau. La phase aqueuse est extraite par 5x20ml de chloroforme puis par 20ml d'éther, puis évaporée à sec. Le résidu est trituré avec 2ml d'eau, le précipité obtenu est recueilli par filtration. On obtient ainsi 370mg du composé n° 15 (rendement 72%).

### Exemple n° 16 : Préparation du composé n° 16 de formule :

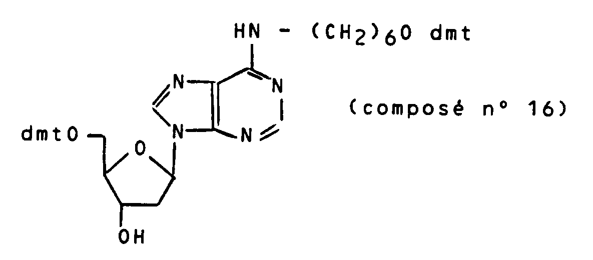

Ce composé est préparé à partir du composé n° 15 en suivant le protocole décrit pour la préparation des composés n° 2 et 13 dans les exemples 2 et 13. Le produit est purifié par chromatographie sur colonne par le procédé décrit dans l'exemple n° 2.

### Exemple 17 : Préparation du composé n° 17 de formule :

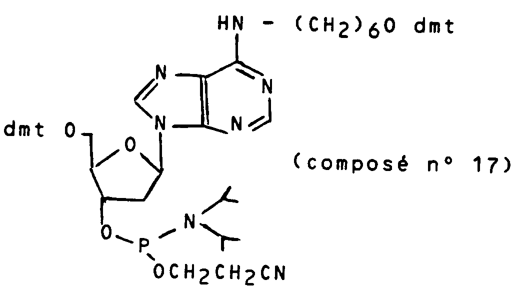

Ce composé est préparé à partir du composé n° 16 en utilisant le procédé de phosphitylation décrit pour les composés 3 et 14.

### Exemple 18 :Préparation d'un oligonucléotide ramifié.

Dans cet exemple on utilise le même procédé que dans l'exemple n° 5 pour préparer un oligonucléotide ramifié correspondant à la séquence suivante : 
5'GCT GTC GGT ACC TAT GAT TC
GCT GTC GGT ACC TAT GAT TC B - GCT CGT ATG TTG TGT GGAAT 3'
dans laquelle A représente le nucléotide formé par l'adénine, C représente le nucléotide formé par la cytosine, G représente le nucléotide formé par la guanine, T représente le nucléotide formé avec la thymine, et B représente l'embranchement formé à partir du composé n° 10 décrit dans l'exemple n° 11.

Pour cette synthèse, on dissout le composé n° 10 dans de l'acétonitrile (à une concentration de 0,15 à 0,2mol/l)et on utilise cette solution dans le cycle standard : "0,2µmol cyanoéthylphosphoramidique" de l'appareil Applied Biosystems utilisé dans l'exemple n° 5.

Le rendement de couplage estimé par mesure du cation diméthoxytrityle est de 94% pour l'introduction du composé n° 10.

### Exemple 19.

On prépare de la même façon que dans l'exemple n° 18 un oligonucléotide complémentaire d'une séquence du phage M13 dont la séquence est la suivante : 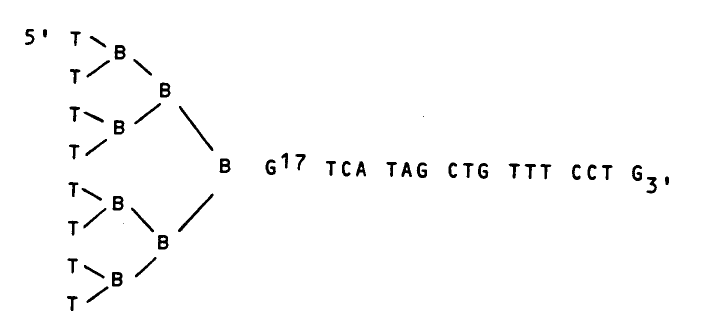
en utilisant les mêmes synthons et le composé n° 10 de l'exemple n° 11 en solution dans l'acétonitrile (0,2mol/l) sur un synthétiseur Applied Biosystem en utilisant la procédure indiquée dans l'exemple 5. Jusqu'à la 17è base, le cycle standard "0,2µmol" est utilisé. Pour les couplages suivants correspondant à 3 branchements désignés par B ci-dessous, ainsi que pour l'introduction de la thymidine, on utilise le cycle standard "1µmol".

### Exemple 20.

Dans cet exemple, on utilise un oligonucléotide ramifié et marqué avec le groupe dinitrophényle (DNP).

On prépare de la même façon que dans l'exemple 5, un oligonucléotide dont la séquence est la suivante : 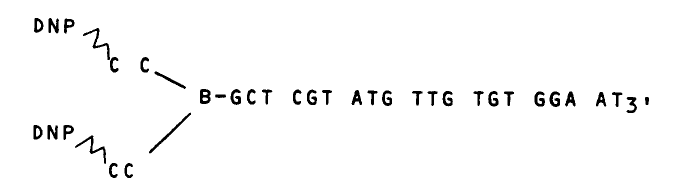
Pour cette préparation, on utilise pour A et G les nucléotides formés respectivement par l'adénine et la guanine protégés sur leur groupement NH₂ exocyclique par des groupes phénoxyacétyle et pour C le nucléotide formé à partir de la cytosine dont le groupe NH₂ exocyclique est protégé par le groupe isobutyryle comme il est décrit par Schulhof et al dans Nucleic Acids Res. 1987, 15, p. 397-416.

Pour B, on utilise le composé n°10 en solution dans l'acétonitrile à 0,2mol/l.

On réalise la synthèse en utilisant le cycle standard "0,2µmol" après le point de branchement obtenu avec le composé n°10 introduit en position 21, on incorpore un résidu cytosine servant d'espaceur, puis un nucléotide marqué en utilisant comme synthon le dérivé phosphoramidite de le 5'-O-(4,4'-diméthoxytrityl)-4-N-(6-N(2,4-dinitrophényl)-aminohexyl)-5-méthyl-2'-désoxycitidine décrit dans le document WO 89/12642.

On a conservé l'oligonucléotide sous sa forme tritylée, puis on l'a soumis à une étape de déprotection dans l'ammoniaque (à 28%) pendant 16h à 20°C.

L'oligonucléotide est gardé sous sa forme tritylée et purifié par chromatographie liquide sous haute pression en phase inverse (R_{T}=48,9 min sur une colonne C-18 gradient 20 à 60% de CH₃CN dans TEAAC 0,05M en 60min, 1ml/min).

L'oligonucléotide se fixe de façon efficace sur des anticorps anti DNP et montre un rapport d'absorbances A₂₆₀/A₃₆₀ de l'ordre de 8,7, ce qui est compatible avec la présence de deux groupes DNP dans la molécule.

## Revendications

1. Dérivé de molécule polyhydroxylée répondant à la formule : 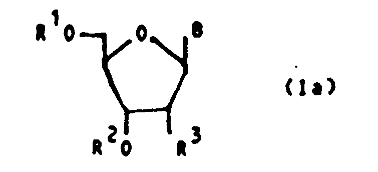 dans laquelle
- R¹ est un radical protecteur d'un groupe OH convenant à la synthèse oligonucléotidique,
- R² est un radical phosphoré adapté à la synthèse oligonucléotidique, non substitué ou substitué par OR¹,
- R³ représente un atome d'hydrogène, le radical hydroxyle, un radical hydroxyle protégé ou le radical R⁴OR¹ avec R¹ ayant la signification donnée ci-dessus et R⁴ représentant un radical hydrocarboné bivalent, linéaire ou ramifié, substitué ou non substitué, comportant éventuellement dans sa chaîne un ou plusieurs groupements choisis parmi O, S, NH, NHCO, NHSO₂, SO₂, CO, -C≡C-, CH=CH, 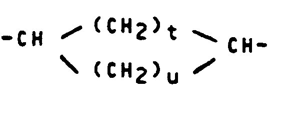 avec t et u étant des nombres entiers de 1 à 2 et 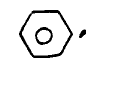 et
- B représente un radical dérivé d'une base purique ou pyrimidique répondant aux formules suivantes : 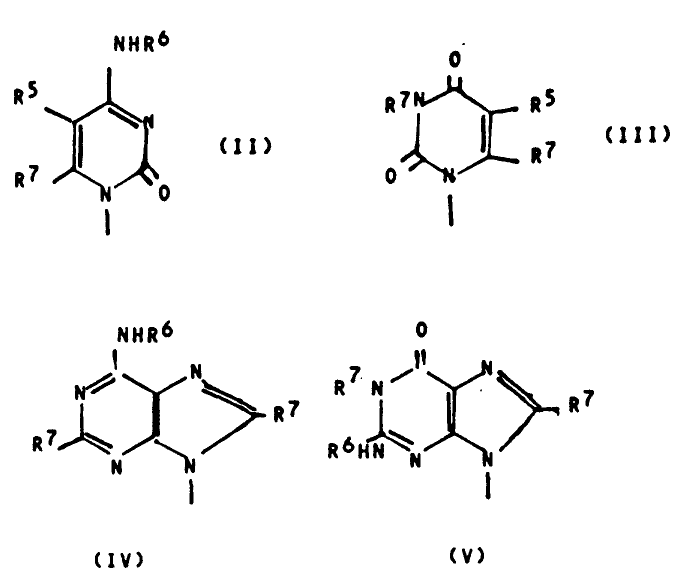 dans lesquelles R⁵ représente H, CH₃, R⁴OR¹ ou R⁸Z, R⁶ représente un groupement protecteur utilisable en synthèse nucléotidique, le radical R⁴OR¹ ou R⁸Z, R⁷ représente un atome d'hydrogène, le radical R⁴OR¹ ou R⁸Z, à condition que l'un au moins des R⁵, R⁶ et R⁷ représente R⁴OR¹ lorsque R³ ne représente pas R⁴OR¹,et que R² ne comporte pas de substituant R⁴OR¹, les R¹ et R⁴ pouvant être identiques ou différents,
- R⁴ représente un radical hydrocarboné bivalent, linéaire ou ramifié, substitué ou non substitué, comportant éventuellement dans sa chaîne un ou plusieurs groupements choisis parmi O, S, NH, NHCO, NHSO₂, SO₂, CO, C≡C, CH=CH, 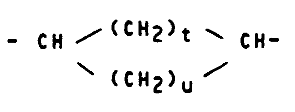 avec t et u étant des nombres entiers de 1 à 2, et 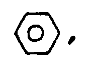
- R⁸ est une simple liaison ou un radical hydrocarboné linéaire ou ramifié, substitué ou non substitué, comportant éventuellement dans sa chaîne et/ou dans ses ramifications un ou plusieurs groupements choisis parmi O, S, NH, NHCO, NHSO₂, SO₂, CO, C≡C, CH=CH, 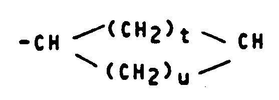 avec t et u étant des nombres entiers de 1 à 2, et 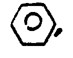 et
- Z est un radical dérivé d'une molécule de marquage, d'une molécule d'un précurseur de marqueur ou d'une molécule capable de s'associer à d'autres molécules, à condition que lorsque B représente le radical de formule (II) avec R⁶ représentant R⁴OR¹, R⁴ ne représente pas (CH₂)ₙ avec n étant un nombre entier de 1 à 6.

2. Dérivé de molécule polyhydroxylée répondant à la formule : 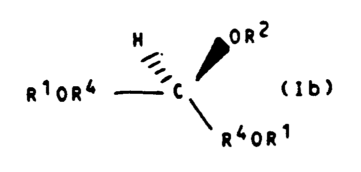 dans laquelle
- R¹ est un radical protecteur d'un groupe OH convenant à la synthèse oligonucléotidique,
- R² est un radical phosphoré adapté à la synthèse oligonucléotidique, non substitué ou substitué par OR¹
- R⁴ représente un radical hydrocarboné bivalent, linéaire ou ramifié, substitué ou non substitué, comportant éventuellement dans sa chaîne un ou plusieurs groupements choisis parmi O, S, NH, NHCO, NHSO₂, SO₂, CO, C≡C, CH=CH, 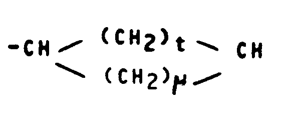 avec t et u étant des nombres entiers de 1 à 2, et

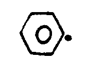

3. Dérivé selon l'une quelconque des revendications 1 et 2, caractérisé en ce que R¹ représente un radical trityle de formule : 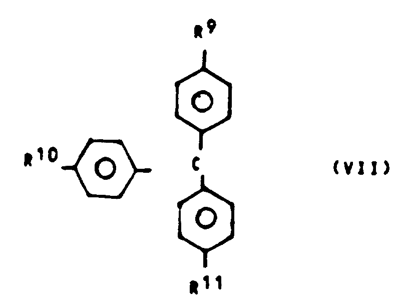 dans laquelle R⁹, R¹⁰ et R¹¹ qui peuvent être identiques ou différents, représentent un atome d'hydrogène, un radical alkyle, alcoxy ou amino.

4. Dérivé selon l'une quelconque des revendications 1 et 2, caractérisé en ce que R² est un radical phosphoré choisi parmi les radicaux de formule : 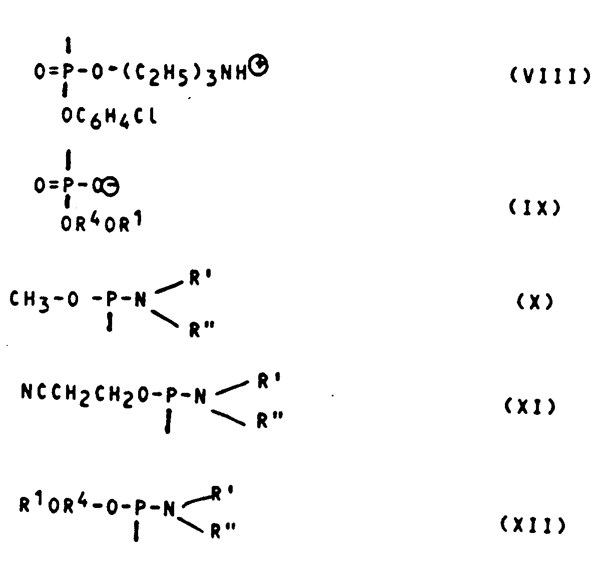 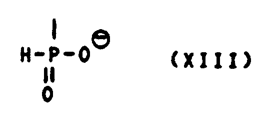 dans lesquelles R¹ et R⁴ ont la signification donnée dans la revendication 1 , R' et R'' pris séparément représentent chacun un radical alkyle, aralkyle ou cycloalkyle contenant jusqu'à 10 atomes de carbone, ou R' et R'' forment ensemble une chaîne alkylène contenant jusqu'à 5 atomes de carbone dans la chaîne principale et un total allant jusqu'à 10 atomes de carbone, ou forment ensemble avec l'atome d'azote un hétérocycle saturé pouvant contenir un ou plusieurs autres hétéroatomes choisis parmi N, O et S.

5. Dérivé selon la revendication 4, caractérisé en ce que R² représente le radical de formule :

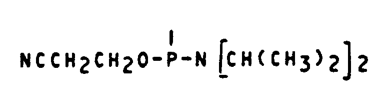

6. Dérivé selon la revendication 5, caractérisé en ce que R¹ est le radical diméthoxytrityle.

7. Dérivé de nucléoside de formule (Ia) selon la revendication 1, caractérisé en ce que B représente :

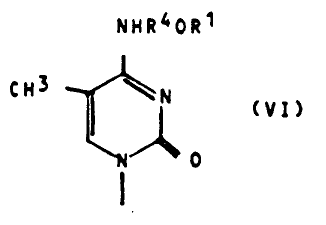

8. Dérivé de nucléoside selon la revendication 7, caractérisé en ce que R⁴ représente -(CH₂)ₙ--NH-(CH₂)ₚ avec n et p étant des nombres entiers de 1 à 6.

9. Dérivé selon la revendication 8, caractérisé en ce que n et p sont égaux à 2.

10. Dérivé selon l'une quelconque des revendications 1, 7, 8 et 9, caractérisé en ce que R³ représente un atome d'hydrogène.

11. Dérivé selon la revendication 2, caractérisé en ce que R⁴ représente -(CH₂)ₙ- avec n étant un nombre entier de 1 à 6.

12. Dérivé selon la revendication 11, caractérisé en ce que n est égal à 3.

13. Procédé pour former au moins une ramification sur un oligonucléotide, caractérisé en ce qu'il comprend les étapes successives suivantes :
a) fixation sur cet oligonucléotide d'un dérivé de molécule polyhydroxylée de formule : 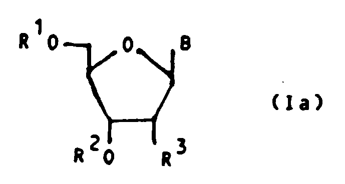 dans laquelle R¹, R², R³ et B ont la signification donnée dans la revendication 1, et
b) réaction des fonctions OH du dérivé fixé avec des molécules capables de réagir avec les fonctions OH obtenues par coupure des liaisons OR¹.

14. Procédé pour former au moins une ramification sur un oligonucléotide, caractérisé en ce qu'il comprend les étapes successives suivantes :
a) fixation sur cet oligonucléotide d'un dérivé de molécule polyhydroxylée de formule : 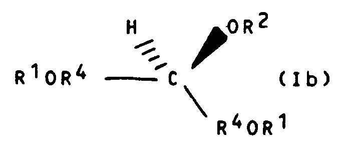 dans laquelle R¹, R² et R⁴ ont la signification donnée dans la revendication 2, et
b) réaction des fonctions OH du dérivé fixé avec des molécules capables de réagir avec les fonctions OH obtenues par coupure des liaisons OR¹.

15. Procédé pour former au moins une ramification sur un oligonucléotide linéaire, caractérisé en ce qu'il comprend les étapes successives suivantes :
a) fixation sur cet oligonucléotide linéaire d'une molécule servant d'espaceur,
b) fixation sur cette molécule servant d'espaceur d'un dérivé de molécule polyhydroxylée de formule : 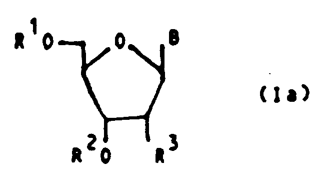 dans laquelle R¹, R², R³ et B ont la signification donnée dans la revendication 1, et
c) réaction des fonctions OH du dérivé fixé avec des molécules capables de réagir avec les fonctions OH obtenues par coupure des liaisons OR¹.

16. Procédé pour former au moins une ramification sur un oligonucléotide linéaire, caractérisé en ce qu'il comprend les étapes successives suivantes :
a) fixation sur cet oligonucléotide linéaire d'une molécule servant d'espaceur,
b) fixation sur cette molécule servant d'espaceur d'un dérivé de molécule polyhydroxylé de formule : 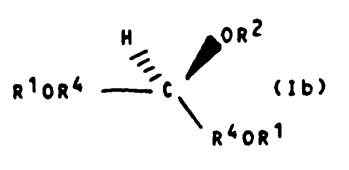 dans laquelle R¹, R² et R⁴ ont la signification donnée dans la revendication 2, et
c) réaction des fonctions OH du dérivé fixé avec des molécules capables de réagir avec les fonctions OH obtenues par coupure des liaisons OR¹.

17. Procédé selon l'une quelconque des revendications 13 à 16, caractérisé en ce que les molécules capables de réagir avec les fonctions OH obtenues par coupure des OR¹ sont choisies parmi les nucléosides, les nucléosides marqués, les molécules pouvant servir d'espaceurs, les molécules de marquage, les molécules capables d'être marquées, les dérivés de molécules polyhydroxylées de formule (Ia) ou (Ib) et les molécules capables de s'associer à d'autres molécules.

18. Procédé selon la revendication 17, caractérisé en ce que la molécule capable d'être marquée est un dérivé de nucléoside que l'on marque ensuite au phosphore 32.

19. Procédé selon la revendication 17, caractérisé en ce que la molécule de marquage est une molécule capable de s'associer à l'avidine ou la streptavidine.

20. Procédé selon la revendication 17, caractérisé en ce que la molécule de marquage est une molécule possédant des propriétés fluorescentes ou luminescentes.

21. Oligonucléotide répondant à la formule :
Aₙ₁-N'ₐ-N-C
dans laquelle :
- n1 est un nombre entier supérieur à 1,
- les A sont des nucléotides identiques ou différents,
- N' est une molécule servant d'espaceur,
- a est égal à 0 ou est un nombre entier,
- N est un dérivé de molécule polyhydroxylée selon la revendication 1 ou 2, et
- C représente l'extrémité de l'oligonucléotide qui comprend au moins deux ramifications identiques ou différentes, formées chacune d'une ou plusieurs molécules identiques ou différentes choisies parmi N', N, M et A avec M représentant un nucléotide marqué, une molécule de marquage ou une molécule d'un précurseur de marqueur ou une molécule capable de s'associer à d'autres molécules.

## Claims

1. Polyhydroxyl molecule derivative in accordance with formula: 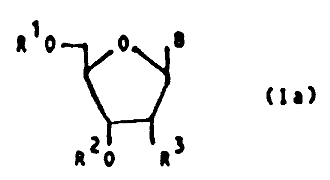 in which
- R¹ is a protective radical of a OH group appropriate for oligonucleotide synthesis,
- R² is a phosphorus radical suitable for oligonucleotide synthesis, which is not substituted or substituted by OR¹,
- R³ represents a hydrogen atom, the hydroxyl radical, the protected hydroxyl radical or the radical R⁴OR¹ with R¹ having the meaning given hereinbefore and R⁴ representing a substituted or unsubstituted, straight or branched, divalent hydrocarbon radical optionally having in its chain one or more groupings chosen from among O, S, N, NHCO, NHSO₂, SO₂, CO, -C≡C-, CH=CH, 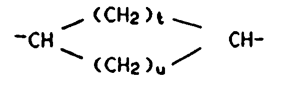 with t and u being integers from 1 to 2 and 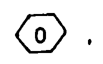 and
- B represents a radical derived from a purine or pyrimidine base in accordance with the following formulas: 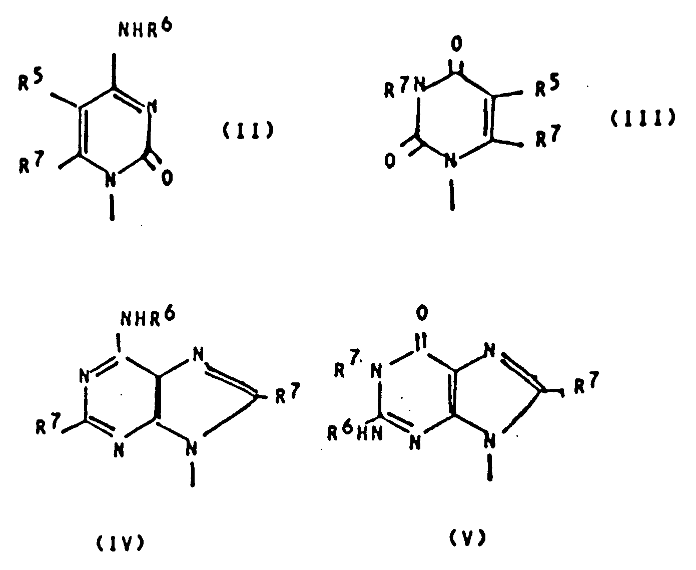 in which R⁵ represents H, CH₃, R⁴OR¹ or R⁸Z, R⁶ represents a protective grouping usable in nucleotide synthesis, the radical R⁴OR¹ or R⁸Z, R⁷ represents a hydrogen atom, the radical R⁴OR¹ or R⁸Z, provided that at least one of the R⁵, R⁶ and R⁷ represents R⁴OR¹ when R³ does not have R⁴OR¹ and R² has no substituent R⁴OR¹, whereby the R¹ and R⁴ can be the same or different,
- R⁴ represents a substituted or unsubstituted, straight or branched, divalent hydrocarbon radical optionally having in its chain one or more groupings chosen from among O, SS, NH, NHCO, NHSO₂, SO₂, CO, C C, CH CH, 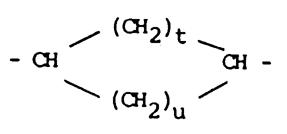 with t and u being integers from 1 to 2 and 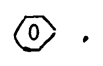
- R⁸ is a single bond or a substituted or unsubstituted, straight or branched, hydrocarbon radial optionally having in its chain and/or in its branchings one or more groupings chosen from among O, S, NH, NHCO, NHSO₂, SO₂, CO, C=C, CH=CH, 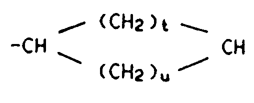 with t and u being integers from 1 to 2 and 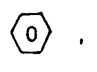 and
- Z is a radical derived from a marking or labeling molecule, a molecule of a marking precursor or a molecule able to associate with other molecules, provided that when B represents the radical of formula (II) with R⁶ representing R⁴OR¹, R⁴ does not represent (CH₂)ₙ with n being an integer from 1 to 6.

2. Polyhydroxyl molecule derivative according to formula: 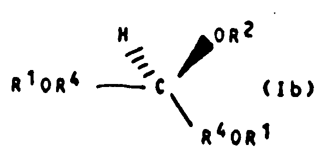 in which
- R¹ is a protective radical of an OH group suitable for oligonucleotide synthesis,
- R² is a phosphorus radical suitable for oligonucleotide synthesis, which is not substituted or substituted by OR¹,
- R⁴ represents a substituted or unsubstituted, straight or branched, divalent hydrocarbon radical, optionally having in its chain one or more groupings chosen from among O, S, NH, NHCO, NHSO₂, SO₂, CO, C≡C, CH=CH, 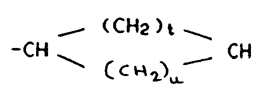 with t and u being integers from 1 to 2 and

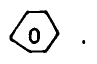

3. Derivative according to either of the claims 1 and 2, characterized in that R¹ represents a trityl radical of formula: 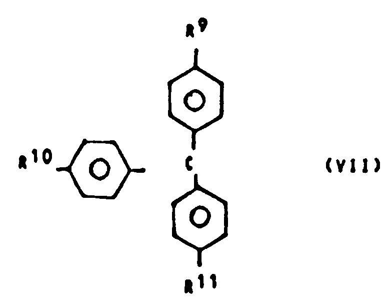 in which R⁹, R¹⁰ and R¹¹, which can be the same or different, represent a hydrogen atom, an alkyl, alkoxy or amino radical.

4. Derivative according to either of the claims 1 and 2, characterized in that R² is a phosphorus radical chosen from among the radicals of formula: 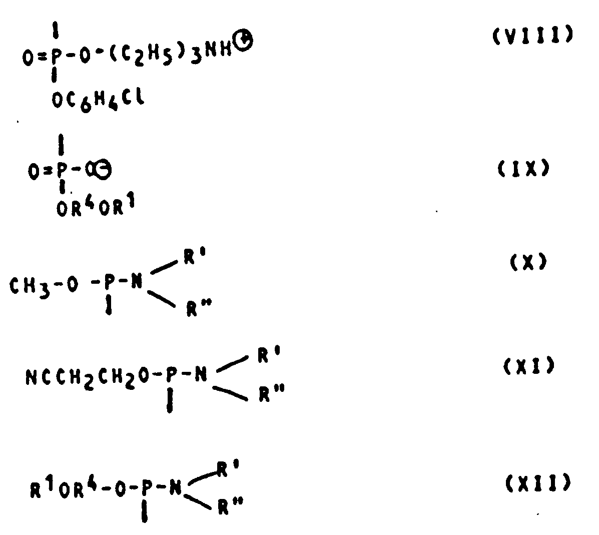 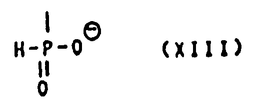 in which R¹ and R⁴ have the meanings given in claim 1, R' and R'', considered separately, each represent an alkyl, aralkyl or cycloalkyl radical containing up to 10 carbon atoms, or R' and R'' together from an alkylene chain containing up to 5 carbon atoms in the main chain and in all up to 10 carbon atoms, or form together with the nitrogen atom a saturated heterocycle able to contain one or more other heteroatoms chosen from among N, O and S.

5. Derivative according to claim 4, characterized in that R² represents the radical of formula:

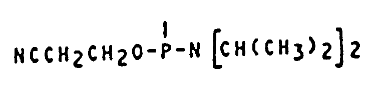

6. Derivative according to claim 5, characterized in that R¹ is the dimethoxytrityl radical.

7. Nucleoside derivative of formula (Ia) according to claim 1, characterized in that B represents:

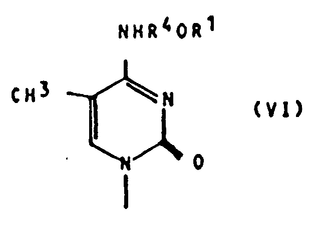

8. Nucleoside derivative according to claim 7, characterized in that R⁴ represents -(CH₂)ₙ-
-NH-(CH₂)ₚ with n and p being integers from 1 to 6.

9. Derivative according to claim 8, characterized in that n and p are equal to 2.

10. Derivative according to any one of the claims 1, 7, 8 and 9, characterized in that R³ represents a hydrogen atom.

11. Derivative according to claim 2, characterized in that R⁴ represents -(CH₂)ₙ- with n being an integer from 1 to 6.

12. Derivative according to claim 11, characterized in that n is equal to 3.

13. Process for forming at least one branching on an oligonucleotide, characterized in that it involves the following successive stages:
a) fixing to said oligonucleotide of a polyhydroxyl molecule derivative of formula: 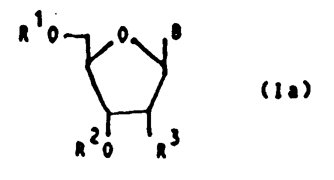 in which R¹, R², R³ and B have the meanings given in claim 1 and
b) reaction of the OH functions of the fixed derivative with molecules able to react with the OH functions obtained by cutting off the OR¹ bonds.

14. Process for forming at least one branching on an oligonucleotide, characterized in that it involves the following successive stages:
a) fixing to said oligonucleotide of a polyhydroxyl molecule derivative of formula: 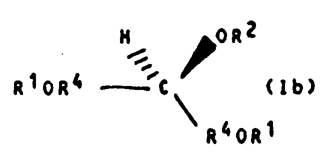 in which R¹, R² and R⁴ have the meanings given in claim 2, and
b) reaction of the OH functions of the fixed derivatives with molecules able to react with the OH functions obtained by cutting off the OR¹ bonds.

15. Process for forming at least one branching on a linear oligonucleotide, characterized in that it involves the following successive stages:
a) fixing to said linear oligonucleotide of a molecule serving as a spacer,
b) fixing to said molecule serving as a spacer of a polyhydroxyl molecule derivative of formula: 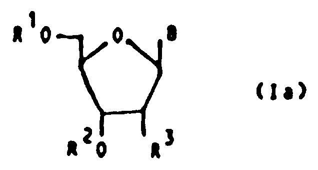 in which R¹, R², R³ and B have the meaning given in claim 1, and
c) reaction of the OH functions of the fixed derivative with molecules able to react with the OH functions obtained by cutting off the OR¹ bonds.

16. Process for forming at least one branching on a linear nucleotide, characterized in that it involves the following successive stages:
a) fixing to said linear oligonucleotide a molecule serving as a spacer,
b) fixing to said molecule serving as a spacer of a polyhydroxyl molecule derivate of formula: 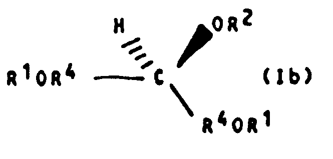 in which R¹, R² and R⁴ have the meanings given in claim 2, and
c) reaction of the OH functions of the fixed derivative with the molecules able to react with the OH functions obtained by cutting off the the OR¹ bonds.

17. Process according to any one of the claims 13 to 16, characterized in that the molecules able to react with the OH functions obtained by cutting off the OR¹ are chosen from among nucleosides, marked nucleosides, molecules able to serve as spacers, marking molecules, molecules able to be marked, derivatives of polyhydroxyl molecules of formula (Ia) or (Ib) and molecules able to associate with other molecules.

18. Process according to claim 17, characterized in that the molecule which can be marked is a nucleoside derivative then marked with phosphorus 32.

19. Process according to claim 17, characterized in that the marking molecule is a molecule able to associate with avidine or streptavidine.

20. Process according to claim 17, characterized in that the marking molecule is a molecule having fluorescent or luminescent properties.

21. Oligonucleotide in accordance with the formula:
Aₙ₁-N'ₐ-N-C
in which:
- n¹ is an integer greater than 1,
- A are identical or different nucleotides,
- N' is a molecule serving as a spacer,
- a is equal to 0 or is an integer,
- N is a polyhydroxyl molecule derivative according to claims 1 or 2, and
- C represents the end of the oligonucleotide having at least two identical or different branchings, each formed from one or more identical or different molecules chosen from among N', N, M and A with M representing a marked nucleotide, a marking molecule or a molecule of a marking precursor or a molecule able to associate with other molecules.

## Patentansprüche

1. Derivat eines polyhydroxylierten Moleküls entsprechend der Formel 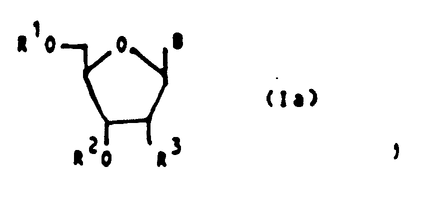 worin
- R¹ eine zur Oligonucleotidsynthese geeignete Schutzgruppe für die OH-Gruppe ist,
- R² ein der Oligonucleotidsynthese angepaßter, phosphorhaltiger, unsubstituierter oder mit OR¹ substituierter Rest ist,
- R³ ein Wasserstoffatom, die Hydroxylgruppe, eine geschützte Hydroxylgruppe oder den Rest R⁴OR¹ bedeutet, wobei R¹ die oben gegebene Bedeutung hat und R⁴ einen zweiwertigen, linearen oder verzweigten, substituierten oder unsubstituierten Kohlenwasserstoffrest bedeutet, der gegebenenfalls in seiner Kette eine oder mehrere Gruppierungen aufweist, die unter O, S, NH, NHCO, NHSO₂, SO₂, CO, -C≡C-, CH=CH, 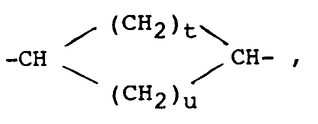 wobei t und u ganze Zahlen von 1 bis 2 sind, und 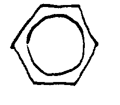 ausgewählt sind, und
- B einen von einer Purin- oder Pyrimidinbase abgeleiteten Rest entsprechend den folgenden Formeln bedeutet: 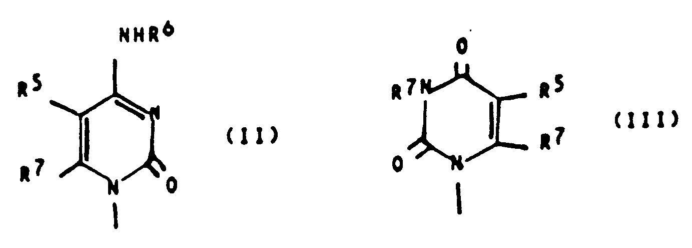 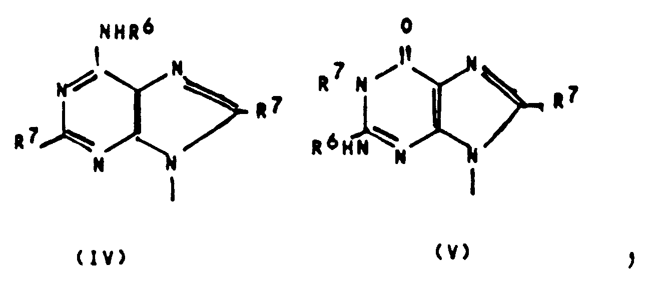 worin R⁵ für H, CH₃, R⁴OR¹ oder R⁸Z steht, R⁶ eine bei der Nucleotidsynthese verwendbare Schutzgruppe oder den Rest R⁴OR¹ oder R⁸Z bedeutet, R⁷ ein Wasserstoffatom oder den Rest R⁴OR¹ oder R⁸Z bedeutet, unter der Bedingung, daß wenigstens einer der Reste R⁵, R⁶ und R⁷ für R⁴OR¹ steht, wenn R³ nicht für R⁴OR¹ steht, und daß R² nicht den Substituenten R⁴OR¹ enthält, während die Reste R¹ und R⁴ gleich oder verschieden sein können,
- R⁴ einen zweiwertigen, linearen oder verzweigten, substituierten oder unsubstituierten Kohlenwasserstoffrest bedeutet, der gegebenenfalls in seiner Kette eine oder mehrere Gruppierungen aufweist, die unter O, S, NH, NHCO, NHSO₂, SO₂, CO, -C≡C-, CH=CH, 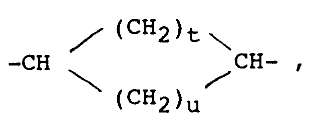 wobei t und u ganze Zahlen von 1 bis 2 sind, und 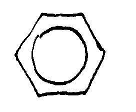 ausgewählt sind,
- R⁸ eine einfache Bindung oder ein linearer oder verzweigter, substituierter oder unsubstituierter Kohlenwasserstoffrest ist, der gegebenenfalls in seiner Kette und/oder in seinen Verzweigungen eine oder mehrere Gruppierungen aufweist, die unter O, S, NH, NHCO, NHSO₂, SO₂, CO, -C≡C-, CH=CH, 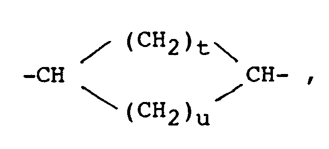 wobei t und u ganze Zahlen von 1 bis 2 sind, und 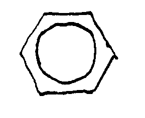 ausgewählt sind, und
- Z ein von einem Markierungsmolekül, von einem Marker-Vorläufermolekül oder von einem Molekül, das imstande ist, sich mit anderen Molekülen zu assoziieren, abgeleiteter Rest ist, unter der Bedingung, daß, wenn B den Rest der Formel (II) bedeutet, worin R⁴OR¹ bedeutet, R⁴ nicht (CH₂)ₙ bedeutet, worin n eine ganze Zahl von 1 bis 6 ist.

2. Derivat eines polyhydroxylierten Moleküls entsprechend der Formel 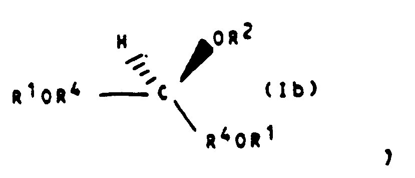 worin
- R¹ eine für die Oligonucleotidsynthese geeignete Schutzgruppe für die OH-Gruppe ist,
- R² ein der Oligonucleotidsynthese angepaßter, phosphorhaltiger, unsubstituierter oder mit OR¹ substituierter Rest ist,
- R⁴ einen zweiwertigen, linearen oder verzweigten, substituierten oder unsubstituierten Kohlenwasserstoffrest bedeutet, der gegebenenfalls in seiner Kette eine oder mehrere Gruppierungen aufweist, die unter O, S, NH, NHCO, NHSO₂, SO₂, CO, -C≡C-, CH=CH, 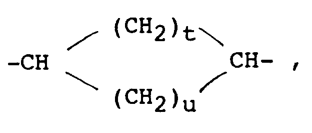 wobei t und u ganze Zahlen von 1 bis 2 sind, und 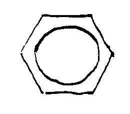 ausgewählt sind.

3. Derivat nach einem der Ansprüche 1 und 2, dadurch gekennzeichnet, daß R¹ einen Tritylrest der Formel 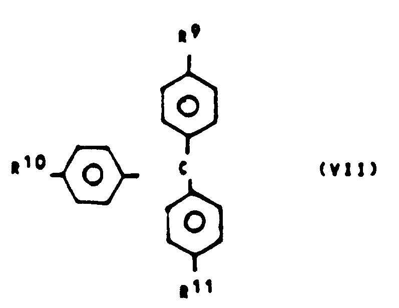 bedeutet, worin R⁹, R¹⁰ und R¹¹, die gleich oder verschieden sein können, ein Wasserstoffatom oder eine Alkyl-, Alkoxy- oder Aminogruppe bedeuten.

4. Derivat nach einem der Ansprüche 1 und 2, dadurch gekennzeichnet, daß R² ein phosphorhaltiger Rest ist, der unter den Resten der Formeln 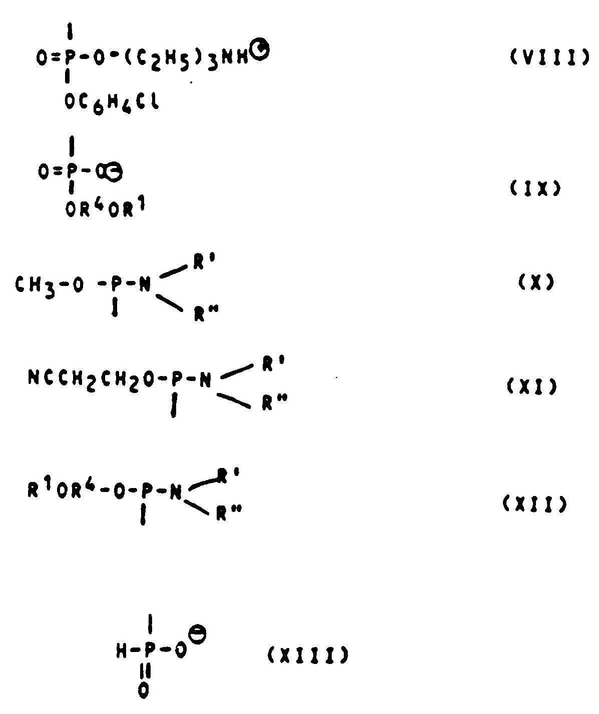 ausgewählt ist, worin R¹ und R⁴ die in Anspruch 1 gegebene Bedeutung haben, R' und R'', für sich genommen, jeweils einen Alkyl-, Aralkyl- oder Cycloalkylrest bedeuten, der bis zu 10 Kohlenstoffatome enthält, oder R' und R'' zusammen eine Alkylenkette bilden, die in der Hauptkette bis zu 5 Kohlenstoffatome und insgesamt bis zu 10 Kohlenstoffatome enthält, oder R' und R'' zusammen mit dem Stickstoffatom einen gesättigten Heterocyclus bilden, der ein oder mehrere unter N, O und S ausgewählte Heteroatome enthalten kann.

5. Derivat nach Anspruch 4, dadurch gekennzeichnet, daß R² den Rest der Formel 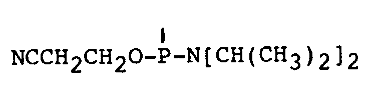 bedeutet.

6. Derivat nach Anspruch 5, dadurch gekennzeichnet, daß R¹ der Dimethoxytritylrest ist.

7. Nucleosidderivat der Formel (Ia) nach Anspruch 1, dadurch gekennzeichnet, daß B 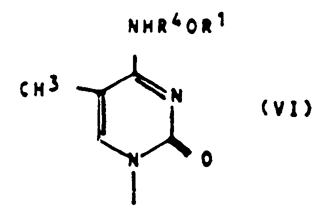 bedeutet.

8. Nucleosidderivat nach Anspruch 7, dadurch gekennzeichnet, daß R⁴ für -(CH₂)ₙ-NH-(CH₂)ₚ steht, worin n und p ganze Zahlen von 1 bis 6 sind.

9. Derivat nach Anspruch 8, dadurch gekennzeichnet, daß n und p gleich 2 sind.

10. Derivat nach einem der Ansprüche 1, 7, 8 und 9, dadurch gekennzeichnet, daß R³ ein Wasserstoffatom bedeutet.

11. Derivat nach Anspruch 2, dadurch gekennzeichnet, daß R⁴ für -(CH₂)ₙ- steht, wobei n eine ganze Zahl von 1 bis 6 ist.

12. Derivat nach Anspruch 11, dadurch gekennzeichnet, daß n gleich 3 ist.

13. Verfahren zur Bildung wenigstens einer Verzweigung in einem Oligonucleotid, dadurch gekennzeichnet, daß es nacheinander die folgenden Schritte umfaßt:
a) Fixieren eines Derivats des polyhydroxylierten Moleküls der Formel 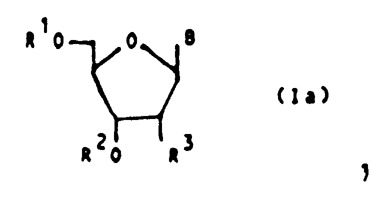 worin R¹, R², R³ und B die in Anspruch 1 gegebene Bedeutung haben, an diesem Oligonucleotid, und
b) Reaktion der OH-Gruppen des fixierten Derivats mit Molekülen, die imstande sind, mit den durch Trennen der OR¹-Bindungen erhaltenen OH-Gruppen zu reagieren.

14. Verfahren zur Bildung wenigstens einer Verzweigung in einem Oligonucleotid, dadurch gekennzeichnet, daß es nacheinander die folgenden Schritte umfaßt:
a) Fixieren eines Derivats des polyhydroxylierten Moleküls der Formel 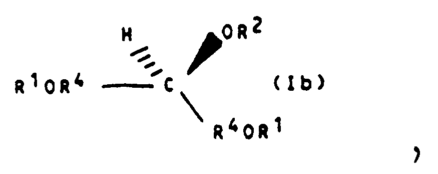 worin R¹, R² und R⁴ die in Anspruch 2 gegebene Bedeutung haben, an diesem Oligonucleotid, und
b) Reaktion der OH-Gruppen des fixierten Derivats mit Molekülen, die imstande sind, mit den durch Trennen der OR¹-Bindungen erhaltenen OH-Gruppen zu reagieren.

15. Verfahren zur Bildung wenigstens einer Verzweigung in einem linearen Oligonucleotid, dadurch gekennzeichnet, daß es nacheinander die folgenden Schritte umfaßt:
a) Fixieren eines als Abstandshalter dienenden Moleküls an diesem Oligonucleotid,
b) Fixieren eines Derivats des polyhydroxylierten Moleküls der Formel 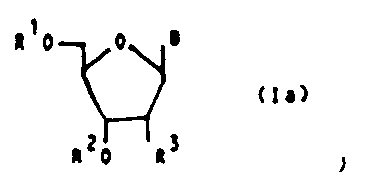 worin R¹, R², R³ und B die in Anspruch 1 gegebene Bedeutung haben, an diesem als Abstandshalter dienenden Molekül,
c) Reaktion der OH-Gruppen des fixierten Derivats mit Molekülen, die imstande sind, mit den durch Trennen der OR¹-Bindungen erhaltenen OH-Gruppen zu reagieren.

16. Verfahren zur Bildung wenigstens einer Verzweigung in einem linearen Oligonucleotid, dadurch gekennzeichnet, daß es nacheinander die folgenden Schritte umfaßt:
a) Fixieren eines als Abstandshalter dienenden Moleküls an diesem linearen Oligonucleotid,
b) Fixieren eines Derivats eines polyhydroxylierten Moleküls der Formel 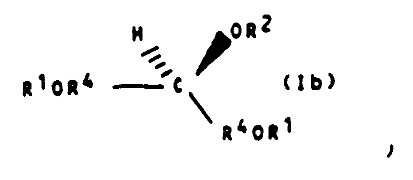 worin R¹, R² und R⁴ die in Anspruch 2 gegebene Bedeutung haben, an diesem als Abstandshalter dienenden Molekül, und
c) Reaktion der OH-Gruppen des fixierten Derivats mit Molekülen, die imstande sind, mit den durch Trennen der OR¹-Bindungen erhaltenen OH-Gruppen zu reagieren.

17. Verfahren nach einem der Ansprüche 13 bis 16, dadurch gekennzeichnet, daß die Moleküle, die imstande sind, mit den durch Trennen der OR¹ erhaltenen OH-Gruppen zu reagieren, unter den Nucleosiden, den markierten Nucleosiden, den Molekülen, die als Abstandshalter dienen können, den Markierungsmolekülen, den Molekülen, die markiert werden können, den Derivaten der polyhydroxylierten Moleküle der Formel (Ia) oder (Ib) und den Molekülen, die sich mit anderen Moleküle assoziieren können, ausgewählt werden.

18. Verfahren nach Anspruch 17, dadurch gekennzeichnet, daß das Molekül, das markiert werden kann, ein Nucleosidderivat ist, das man dann mit Phosphor-32 markiert.

19. Verfahren nach Anspruch 17, dadurch gekennzeichnet, daß das Markierungsmolekül ein Molekül ist, das sich mit Avidin oder Streptavidin assoziieren kann.

20. Verfahren nach Anspruch 17, dadurch gekennzeichnet, daß das Markierungsmolekül ein Molekül ist, das Fluoreszenz- oder Lumineszenzeigenschaften besitzt.

21. Oligonucleotid entsprechend der Formel
Aₙ₁-N'ₐ-N-C ,
worin
- n1 eine ganze Zahl größer als 1 ist,
- die A gleiche oder verschiedene Nucleotide sind,
- N' ein als Abstandshalter dienendes Molekül ist,
- a gleich 0 oder eine ganze Zahl ist,
- N ein Derivat eines polyhydroxylierten Moleküls nach Anspruch 1 oder 2 ist und
- C das Ende des Oligonucleotids ist, das wenigstens zwei gleiche oder verschiedene Verzweigungen umfaßt, die jeweils aus einem oder mehreren, gleichen oder verschiedenen Molekülen gebildet sind, die unter N', N, M und A ausgewählt sind, wobei M ein markiertes Nucleotid, ein Markierungsmolekül oder ein Marker-Vorläufermolekül oder ein Molekül, das sich mit anderen Molekülen assoziieren kann, bedeutet.
